# EUROPEAN PATENT APPLICATION

(11) **EP 4 375 648 A1**
(43) Date of publication of application: **29.05.2024**
(21) Application number: 22857368.9
(22) Date of filing: 19.05.2022
(51) Int. Cl.: G01N 21/78, C01G 41/02

(54) **HYDROGEN LEAKAGE DETECTING MATERIAL, AND PREPARATION METHOD THEREFOR AND USE THEREOF**

(30) Priority: 19.08.2021 CN 202110955315
(71) Applicant: China Petroleum & Chemical Corporation, Beijing 100728 (CN); Sinopec Research Institute of Safety Engineering Co., Ltd., Qingdao, Shandong 266104 (CN)
(72) Inventor: YANG, Zhe, Qingdao, Shandong 266104 (CN); LIU, Huan, Qingdao, Shandong 266104 (CN); WANG, Lin, Qingdao, Shandong 266104 (CN); TAO, Bin, Qingdao, Shandong 266104 (CN); ZHANG, Jianzhong, Qingdao, Shandong 266104 (CN); LIU, Fangming, Qingdao, Shandong 266104 (CN); WANG, Zhenzhong, Qingdao, Shandong 266104 (CN); ZHAO, Wenqing, Qingdao, Shandong 266104 (CN); DING, Lili, Qingdao, Shandong 266104 (CN); GAO, Jian, Qingdao, Shandong 266104 (CN)
(74) Representative: Hoffmann Eitle
(86) International application number: PCT/CN2022/093814
(87) International publication number: WO 2023/020052

(57) **Abstract**

A hydrogen leakage detecting material, and a preparation method therefor and the use thereof. The hydrogen leakage detecting material comprises a color-changing substrate and an active component, wherein the color-changing substrate is hexagonal-phase tungsten trioxide having a microstructure of a nanorod bundle formed by multiple nanorods, and the active component is noble metal nanoparticles. A starting reduction temperature of the hydrogen leakage detecting material on hydrogen is less than or equal to 100°C. The hydrogen leakage detecting material has a relatively high sensitivity to hydrogen under different conditions, and has significant color change and shorter color change response time.

## Description

### CROSS-REFERENCE TO RELATED APPLICATIONS

The application claims the benefit of Chinese patent application No. "202110955315.1", filed on August 19, 2021, entitled "HYDROGEN LEAKAGE DETECTING MATERIAL, AND PREPARATION METHOD THEREFOR AND USE THEREOF", the content of which is specifically and entirely incorporated herein by reference.

### FIELD

The present disclosure relates to the technical field of gas sensitive material, and particularly discloses a hydrogen leakage detecting material and a preparation method therefor and a use thereof.

### BACKGROUND

Hydrogen gas has many advantages such as clean, environmental friendly, renewable, and high efficiency of energy conversion, and is praised as the "ultimate energy" of the 21st century, and has wide application prospects in vehicle energy supply, energy storage and other fields. In view of the aforesaid advantages of hydrogen energy, the United States of America (USA), Japan, the European Union (EU) and other developed countries and regions have formulated national strategies for developing hydrogen energy, and have vigorously developed hydrogen energy as an important component of future energy structure.

However, safety of hydrogen energy is always an essential base and important premise for developing the hydrogen energy industry. Hydrogen gas is composed of small molecules, which are prone to penetrate the material and leak, and the hydrogen gas is colorless and odorless, such that the leaked hydrogen gas cannot be perceived by human body; in addition, the burning and explosion range of hydrogen gas concentration is wide (4-75%), and the minimum ignition energy is low (0.02 mJ). Therefore, the rapid and efficient detection of hydrogen gas leakage is critical for avoiding the evolution of hydrogen gas leakage incident into more severe hydrogen gas burning and explosion accidents. Currently, indicating hydrogen gas leakage by color change derived from reaction between hydrogen gas and a portion of inorganic or organic materials is a potential technological route to achieve the rapid and efficient detection of hydrogen gas leakage with a high coverage.

Tungsten trioxide is a commonly used gas-chromic material, which undergoes reversible/irreversible color change when contacting with gases, especially hydrogen-containing reducing gases such as hydrogen gas, hydrogen sulfide, ammonia gas, ethyl alcohol. Due to the gas-chromic property of tungsten trioxide, it is generally used as the material for detecting leakage of hydrogen gas, hydrogen sulfide and other gas. Tungsten trioxide is a typical multi-phase compound that exhibits crystalline phases from a low temperature monoclinic phase, a triclinic phase, a room temperature monoclinic phase, a tetragonal phase, and a metastable crystalline phase (e.g., hexagonal phase) along with the temperature change. The hexagonal-phase tungsten trioxide has a six-membered intracrystalline channel structure capable of accommodating H, Li and other ions to free access (High surface area tunnels in hexagonal WO3, Nano Lett. 2015, 15, 7, 4834-4838), and thus has superior performance for hydrogen leakage detection. The researchers have carried out a portion of research work to control synthesis of the nanostructures of tungsten trioxide.

The prior art produces the hydrogen leakage detecting materials capable of quickly indicating hydrogen leakage by introducing a noble metal to the tungsten trioxide.

CN112279302A uses a hydrothermal method to prepare a tungsten trioxide which is doped with a noble metal and has a monoclinic crystal structure. However, tungsten trioxide crystalline phase prepared with the method is a monoclinic phase, instead of a hexagonal-phase showing superior physicochemical activity, and the patent fails to disclose the dispersion condition of noble metal on tungsten trioxide and the hydrogen-sensitive color change property.

CN101318704A discloses a preparation method of electrical hydrogen leakage detecting material, wherein a tungsten trioxide nano-wire having a hexagonal-phase structure is prepared by reacting sodium tungstate with hydrochloric acid and then performing a centrifugal separation, and carrying out a hydrothermal reaction by placing the centrifugal separated substance in a potassium sulfate solution, and the tungsten trioxide nano-wire is further processed to an electrical hydrogen sensor. The patent mainly utilizes the electrical property of tungsten trioxide to detect hydrogen leakage, and does not recognize how to use chemical color change to indicate detection of hydrogen leakage; therefore, the color change is not significant when the tungsten trioxide nano-wire disclosed in the patent encounters hydrogen leakage in practical applications.

CN110132502A discloses an active material capable of indicating the existence of hydrogen gas through color change, the active material comprises an inert substance serving as a substrate and generating no metachromatism with hydrogen gas, a metal oxide attached to the surface of the inert substance and capable of reacting with hydrogen gas, and metal nanoparticles attached to the metal oxide, the active material can quickly generate a color change that can be easily distinguished in the case of contacting with hydrogen gas, and has the characteristics of high response speed and high sensitivity. However, the method does not limit the crystalline phase and the microscopic morphology of the metal oxide, and the metal nanoparticles are attached to the surface of the metal oxide through a co-precipitation method, and easily result in agglomeration of the metal nanoparticles on a surface of the metal oxide.

### SUMMARY

The present disclosure aims to overcome the problems in the prior art concerning the hydrogen leakage detecting material using tungsten trioxide as the color-changing substrates, such as a slow color change speed at room temperature, an insignificant color contrast before and after the color change, and to provide a detecting material capable of rapidly and sensitively detecting hydrogen leakage under various working conditions, and a method for preparing the same.

In order to achieve the above objects, a first aspect of the present disclosure provides a hydrogen leakage detecting material comprising a color-changing substrate and an active component;
wherein the color-changing substrate is hexagonal-phase tungsten trioxide, and has a microstructure of a nanorod bundle formed by stacking multiple nanorods;
the active component is noble metal nanoparticles;
wherein a starting reduction temperature of the hydrogen leakage detecting material on hydrogen gas is less than or equal to 100°C.

A second aspect of the present disclosure provides a method for preparing a hydrogen leakage detecting material comprises the following steps:
(i) carrying out a first mixing for a noble metal precursor, tungsten trioxide and deionized water to obtain a solid-liquid mixture;
(ii) subjecting an organic liquid phase reduction reagent and the solid-liquid mixture to a second mixing to obtain a reaction mixture;
(iii) subjecting the reaction mixture to an organic liquid phase reduction;
wherein the tungsten trioxide is hexagonal-phase tungsten trioxide, and has a microstructure of a nanorod bundle formed by stacking multiple nanorods.

A third aspect of the present disclosure provides a use of the aforementioned hydrogen leakage detecting material in the hydrogen-containing reducing gas leakage detection product.

Due to the technical scheme, the present disclosure produces the favorable effects as follows:
1. The hydrogen leakage detecting material of the present disclosure uses a hexagonal-phase tungsten trioxide, its six-membered intra-crystal channel structure not only has a characteristic suitable for transportation of proton, hydrogen gas and other small molecules, produces a favorable promotion effect on the transportation of active hydrogen after adsorption and dissociation, and the unique nanorod bundle microstructure provides the diffusion and transport channels for hydrogen gas and dissociated hydrogen atoms, thus the present disclosure greatly enhances the color change rate and significantly shortens the color-changing response time by achieving an effective combination of the six-membered intra-crystal channel structure and the nanorod bundle microstructure.
2. The tungsten trioxide with a nanorod bundle microstructure provides an effective carrier structure for the highly uniform dispersion of noble metal nanoparticles, preferably the dispersity of noble metal nanoparticles is larger than or equal to 70%, a minimum size of the noble metal nanoparticles is 2nm, such that a starting reduction temperature of the hydrogen leakage detecting material on hydrogen gas is reduced to below 60°C. In addition, the tungsten trioxide with the structure is white, its reflectivity to visible light within a wavelength range of 400-750nm is larger than or equal to 50%, preferably larger than or equal to 65%, the prepared hydrogen leakage detecting material is more advantageous for observing the color change before and after the introduction of hydrogen gas.
3. The inventors have discovered through researches that the hydrogen leakage detecting material prepared with the tungsten trioxide having the microstructure characteristic (of nanorod bundle) exhibits a higher sensitivity to hydrogen gas, and it has a faster color-changing response time and a more significant color change than the detecting material using the tungsten trioxide having other microstructure under the same conditions.
4. The hydrogen leakage detecting material of the present disclosure has significant change of reflectivity upon contacting with hydrogen gas, it has a reflectivity change difference to visible light within a wavelength range of 400-750nm being larger than or equal to 20%. Compared with the hydrogen leakage detecting material using tungsten oxide as the color-changing substrate in the prior art, a significant color change is achieved without using a light-colored inert substrate material; the hydrogen leakage detecting material has a reflectivity change difference to blue light within the wavelength range of 400-500nm being larger than or equal to 22%, and a reflectivity change difference to red light within the wavelength range of 600-750 nm being larger than or equal to 30%, it produces a significant visual reminder effect. It has a color-changing response time of less than or equal to 2s for pure hydrogen and a color-changing response time of less than or equal to 15s for hydrogen gas having a concentration of 1 vol. % at room temperature, a significant decrease in color-changing response time can facilitates the rapid discovery of the hydrogen leakage site.
5. The hydrogen leakage detecting material of the present disclosure has a faster color-changing response time for hydrogen gas having various concentrations and different flow rates under the different temperatures, it can be applied to different hydrogen leakage detection scenarios.
6. The method for preparing the hydrogen leakage material according to the present disclosure uses an organic liquid phase reduction method, which allows the noble metal precursor to be slowly reduced by adjusting the type of the organic reagent and the reaction temperature, avoiding the agglomeration due to the rapid reduction of the noble metal or the high-temperature process caused by the inorganic reducing reagent and the high-temperature gas phase process, thereby obtaining highly dispersed noble metal nanoparticles, and exerting more excellent gas-sensitive color change property.

### BRIEF DESCRIPTION OF THE DRAWINGS

Fig. 1 shows the X-Ray Diffraction (XRD) graphs of tungsten trioxide A1 prepared in Example 1 and the hydrogen leakage detecting material B1 prepared in Example 4, wherein the reference sign 1 illustrates an XRD graph of the hydrogen leakage detecting material B1, and the reference sign 2 illustrates an XRD graph of the tungsten trioxide A1.
FIG. 2 shows the Temperature Profile Recorder (TPR) analysis results of tungsten trioxide A1 prepared in Example 1 and the hydrogen leakage detecting material B1 prepared in Example 4, wherein the reference sign 1 illustrates the TPR analysis result of the color-changing substrate tungsten trioxide, and the reference sign 2 illustrates the TPR analysis result of hydrogen leakage detecting material.
FIG. 3 shows the Scanning Electron Microscope (SEM) photograph of tungsten trioxide A1 prepared in Example 1.
FIG. 4 shows the Transmission Electron Microscope (TEM) photograph of tungsten trioxide A1 prepared in Example 1.
FIG. 5 shows a High Resolution Transmission Electron Microscope (HRTEM) image of tungsten trioxide A1 prepared in Example 1.
FIG. 6 illustrates a HRTEM image of the hydrogen leakage detecting material B1 prepared in Example 4.
FIG. 7 shows an EDS element distribution photograph of the hydrogen leakage detecting material B1 prepared in Example 4, wherein FIG. A is a TEM image, FIG. B is a distribution chart of the element tungsten, FIG. C is a distribution chart of the element oxygen, and FIG. D is a distribution chart of the element platinum, as can be seen, the elements tungsten, oxygen and platinum are highly uniformly dispersed and the platinum agglomeration is not detected.

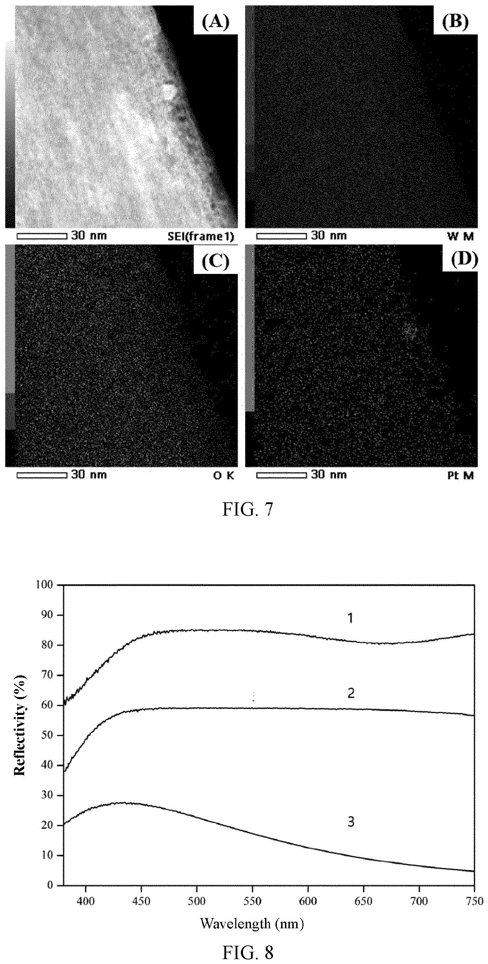

FIG. 8 shows a reflection spectrum of tungsten trioxide A1 prepared in Example 1, and a reflection spectrum of the hydrogen leakage detecting material B1 prepared in Example 4 before and after introducing H₂ having a concentration of 10vol.% at a flow rate of 100mL/min at room temperature, wherein the reference sign 1 represents a reflection spectrum of tungsten trioxide A1 prepared in Example 1, the reference sign 2 represents a reflection spectrum of hydrogen leakage detecting material B1 before introducing the hydrogen gas, and the reference sign 3 represents a reflection spectrum of hydrogen leakage detecting material B1 after introducing the hydrogen gas for 60s.

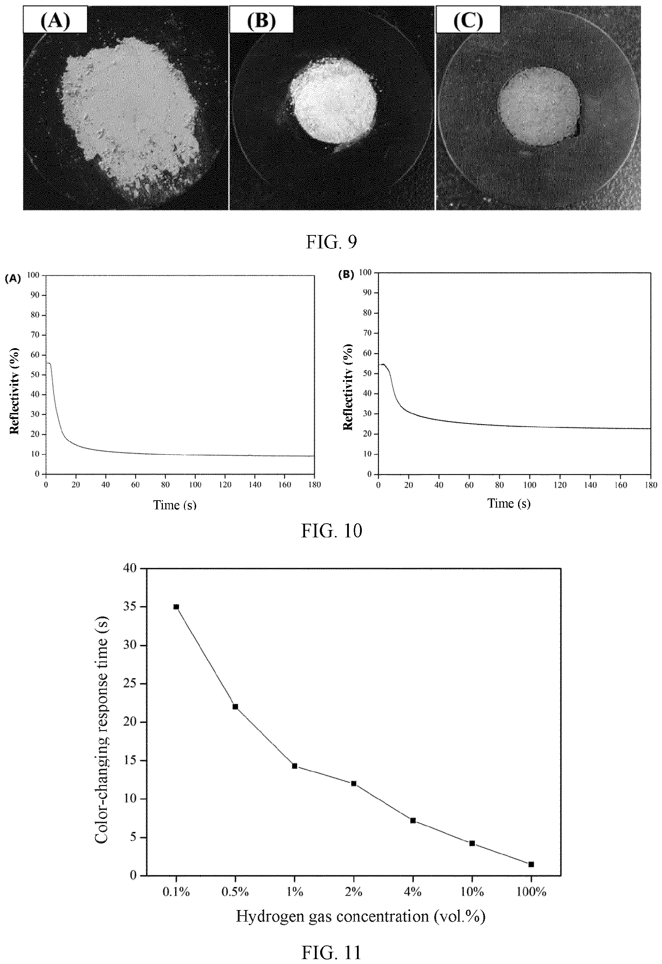

FIG. 9 shows the photograph of tungsten trioxide A1 prepared in Example 1, and a comparison diagram of the photographs of the hydrogen leakage detecting material B1 prepared in Example 1 before and after introducing the hydrogen gas having a concentration of 10vol.% at a flow rate of 100mL/min at room temperature, wherein the reference sign A represents a photograph of tungsten trioxide A1, the reference sign B represents a hydrogen leakage detecting material before introducing the hydrogen gas, and the reference sign C represents a hydrogen leakage detecting material after introducing the hydrogen gas 60s, it has a dark blue color.
FIG. 10 is a graph showing the change of reflectivity of hydrogen leakage detecting material B1 prepared in Example 4 with the time of introducing hydrogen gas having a H₂ concentration of 10vol.% (hydrogen flow rate is 100mL/min, room temperature), the reference sign A illustrates a graph showing the reflectivity change to light at the wavelength of 700nm with the time of introducing hydrogen gas, and the reference sign B illustrates a graph showing the reflectivity change to light at the wavelength of 475nm with the time of introducing hydrogen gas.
FIG. 11 illustrates the color-changing response time of the hydrogen leakage detecting material B1 prepared in Example 4 for the hydrogen gas at various H₂ concentrations (flow rate of 200mL/min, room temperature).
FIG. 12 illustrates the color-changing response time of the hydrogen leakage detecting material B1 prepared in Example 4 for the hydrogen gas at various flow rates (H₂ concentration is 10vol.%, room temperature).
FIG. 13 illustrates the color-changing response time of the hydrogen leakage detecting material B1 prepared in Example 4 for the hydrogen gas at various gas temperatures (flow rate is 200mL/min, H₂ concentration is 10vol.%).
FIG. 14 shows the XRD diagrams of tungsten trioxide DA1 and DA2 prepared in Comparative Example 1 and Comparative Example 2, the reference sign 1 represents the XRD diagram of tungsten trioxide DA1, and the reference sign 2 represents the XRD diagram of tungsten trioxide DA2.
FIG. 15 shows the SEM photograph of tungsten trioxide DA1 prepared in Comparative Example 1.
FIG. 16 shows the SEM photograph of tungsten trioxide DA2 prepared in Comparative Example 2.
FIG. 17 shows the TPR analysis result of hydrogen leakage detecting material DB1 prepared in Comparative Example 3.
FIG. 18 illustrates the photographs before and after introducing hydrogen gas into the hydrogen leakage detecting material DB4 prepared in Comparative Example 6. The reference sign A represents the hydrogen leakage detecting material before introducing hydrogen gas, the color is yellow; the reference sign B represents the hydrogen leakage detecting material after introducing hydrogen gas having a concentration of 10vol.% at a flow rate of 200mL/min at the room temperature for 15 min (900s), the color is also yellow.

### DESCRIPTION OF THE PREFERRED EMBODIMENT

The terminals and any value of the ranges disclosed herein are not limited to the precise ranges or values, such ranges or values shall be comprehended as comprising the values adjacent to the ranges or values. As for numerical ranges, the endpoint values of the various ranges, the endpoint values and the individual point value of the various ranges, and the individual point values may be combined with one another to produce one or more new numerical ranges, which should be deemed have been specifically disclosed herein.

A first aspect of the present disclosure provides a hydrogen leakage detecting material comprising a color-changing substrate and an active component;
wherein the color-changing substrate is hexagonal-phase tungsten trioxide, and has a microstructure of a nanorod bundle formed by stacking multiple nanorods;
the active component is noble metal nanoparticles;
wherein a starting reduction temperature of the hydrogen leakage detecting material on hydrogen gas is less than or equal to 100°C.

In the present disclosure, the term "starting reduction temperature for hydrogen" refers to the starting temperature when an obvious hydrogen-consuming peak occurs in the TPR analysis, as obtained by a chemical adsorption instrument. The lower is the starting reduction temperature for hydrogen gas, the better is the reducing property of the hydrogen leakage detecting material.

In some embodiments of the present disclosure, the nanorod has a diameter within a range of 1-10nm; the length of the nanorod bundle is within a range of 500-1,000nm, the diameter is within a range of 50-100nm; the nanorod bundle contains 20 or more nanorods, and the length-diameter ratio of the nanorod bundle is within a range of 5-20.

In the present disclosure, the microstructure of the hexagonal-phase tungsten trioxide is observed and obtained by the Scanning Electron Microscope (SEM), the High Power Transmission Electron Microscope analysis.

In the present disclosure, the crystalline phase hexagonal phase of the tungsten trioxide is obtained by the XRD diffraction spectrogram.

In some embodiments of the present disclosure, the hexagonal-phase tungsten trioxide has main exposed crystal planes (002), (100) and (001), the XRD characteristic diffraction peak intensities satisfy the following conditions: I₍₀₀₂₎=0.9-1.1×I₍₁₀₀₎, and I₍₀₀₂₎=1.2-2×I₍₀₀₁₎, wherein I₍₀₀₂₎ denotes the intensity of the characteristic diffraction peak corresponding to the (002) crystal plane, I₍₁₀₀₎ denotes the intensity of the characteristic diffraction peak corresponding to the (100) crystal plane, and I₍₁₀₁₎ denotes the intensity of the diffraction peak corresponding to the (001) crystal plane.

Unless otherwise specified in the present disclosure, the XRD diffraction spectrogram is measured and obtained by the X-ray diffractometer.

The inventors have discovered in the researches that the exposure degree of crystal faces is related to the structure and properties of the formed tungsten trioxide. When the tungsten trioxide having the above-mentioned conditions of diffraction peak intensity is used as a color-changing substrate, the produced hydrogen-sensitive color changing material changes color more rapidly when contacting with hydrogen, it is more sensitive to hydrogen gas with different concentrations, temperatures and flow rates.

In some embodiments of the present disclosure, the reflectivity of the hexagonal-phase tungsten trioxide to visible light within a wavelength range of 400-750nm as measured by a spectrometer is larger than or equal to 50%, preferably larger than or equal to 60%, more preferably larger than or equal to 65%, when using a standard that the reflectivity of barium sulfate is equal to 100%. Preferably, when using a standard that the reflectivity of barium sulfate is equal to 100%, the reflectivity of the hexagonal-phase tungsten trioxide to visible light within the wavelength range of 400-750nm measured by a spectrometer may be any value of 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, or the numerical value within a range consisting of any two values thereof. For example, the reflectivity of a hexagonal-phase tungsten trioxide to light in all wavelengths in the 400-750nm wavelength range is the lowest level of 50% to the light with a wavelength of 400nm, and the reflectivity is the highest level of 70% to the light with a wavelength of 700nm, and the reflectivity of the hexagonal-phase tungsten trioxide to visible light within the wavelength range of 400-750nm is within a range of 50-70%.

It should be noted that the term "the reflectivity of said hexagonal-phase tungsten trioxide to light within a wavelength range is greater than or equal to a certain value" refers to that the lowest value of the reflectivity to light within the wavelength range. For example, the reflectivity of the hexagonal-phase tungsten trioxide to light within a wavelength range of 400-750nm, which has a lowest reflectivity of 50% to the light having a wavelength of 400nm, it means that the reflectivity of the hexagonal-phase tungsten trioxide to light within a wavelength of 400-750nm is larger than or equal to 50%. Similarly, the term "the reflectivity of said hexagonal-phase tungsten trioxide to light within a wavelength range is less than or equal to a certain value" refers to that the highest value of the reflectivity to light within the wavelength range.

In some embodiments of the present disclosure, the hexagonal-phase tungsten trioxide has a reflectivity to blue light within the wavelength range of 400-500nm as measured by a spectrometer being larger than or equal to 50% and a reflectivity to red light within the wavelength range of 600-750nm being larger than or equal to 70% when using a standard that the reflectivity of barium sulfate is equal to 100%. Preferably, when using a standard that the reflectivity of barium sulfate is equal to 100%, the reflectivity of the hexagonal-phase tungsten trioxide to blue light within the wavelength range of 400-500nm may be any value of 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, or the numerical value within a range consisting of any two values thereof, and the reflectivity to red light within the wavelength range of 600-750nm may be any value of 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, or the numerical value within a range consisting of any two values thereof. Preferably, when using a standard that the reflectivity of barium sulfate is equal to 100%, the hexagonal-phase tungsten trioxide has a reflectivity to blue light within the wavelength range of 400-500nm being greater than or equal to 67%, and a reflectivity to red light within the wavelength range of 600-750nm being greater than or equal to 72%.

Unless otherwise specified in the present disclosure, the reflectivity is measured by a spectrometer by using a standard that the reflectivity of barium sulfate is equal to 100%. Unless otherwise specified in the present disclosure, "visible light" refers to the light within the wavelength range of 400-750nm; "blue light" refers to the light within the wavelength range of 400-500nm; and "red light" refers to the light within the wavelength range of 600-750nm.

In the present disclosure, the reflectivity refers to a percentage of the reflected light flux for visible light relative to the incident light flux. The reflectivity has corresponding relation with the color and its shade, the higher is the reflectivity to visible light, the lighter is the color; the higher is the reflectivity to light within a certain wavelength range, the higher is the proportion of the color corresponding to the wavelength range in the observed color. In the present disclosure, the reflectivity of blue light within the wavelength range of 400-500nm is from 30% to 37%, and the reflectivity of red light within the wavelength range of 600-750nm is from 20% to 30%. In the present disclosure, the reflectivity of "dark blue color" corresponding to the light within the wavelength range of 400-500nm is 15-29%, and the reflectivity of red light within the wavelength range of 600-750nm is 1-15%.

In some embodiments of the present disclosure, the noble metal nanoparticles are at least one selected from the group consisting of platinum nanoparticles, palladium nanoparticles, rhodium nanoparticles and gold nanoparticles, preferably platinum nanoparticles.

In the present disclosure, the noble metal nanoparticles are highly dispersed on the color-changing substrate. In some embodiments of the present disclosure, the dispersity of the noble metal nanoparticles on the color-changing substrate is larger than or equal to 70%, preferably larger than or equal to 75%, more preferably larger than or equal to 80%. Preferably, the dispersity of the noble metal nanoparticles on the color-changing substrate may be any value of 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100%, or the numerical value within a range consisting of any two values thereof.

In some embodiments of the present disclosure, the average size of the noble metal nanoparticles is less than or equal to 5nm, preferably less than or equal to 2nm.

In the present disclosure, an average size of the noble metal nanoparticles is analyzed and obtained through the High Resolution Transmission Electron Microscope (HRTEM) in combination with the Energy Dispersive Spectrometer (EDS).

In the present disclosure, the dispersity refers to the dispersion performance of noble metal nanoparticles on the color-changing substrate as measured by the quantitative hydrogen pulse chemical reaction method.

In some embodiments of the present disclosure, the noble metal nanoparticles are contained in an amount of 0.1-1.5wt.%, based on the total mass of the hydrogen leakage detecting material.

Preferably, the amount of the noble metal nanoparticles based on the total mass of the hydrogen leakage detecting material may be any value of 0.1 wt. %, 0.2wt.%, 0.3wt.%, 0.4 wt.%, 0.5 wt.%, 0.6 wt.%, 0.7wt.%, 0.8 wt.%, 0.9wt.%, 1wt.%, 1.1 wt.%, 1.2wt.%, 1.3wt.%, 1.4wt.%, 1.5wt.%, or the numerical value within a range consisting of any two values thereof.

In the present disclosure, the amount of said noble metal nanoparticles is detected by the X-ray fluorescence method.

In some embodiments of the present disclosure, a starting reduction temperature of the hydrogen leakage detecting material on hydrogen gas is less than or equal to 80°C, preferably less than or equal to 60°C. Preferably, the starting reduction temperature of the hydrogen leakage detecting material on hydrogen gas may be any value of 100°C, 95°C, 90°C, 85°C, 80°C, 75°C, 70°C, 65°C, 60°C, 55°C, 50°C, 45°C, 40°C, 35°C, 30°C, 25°C, 20°C, 15°C, 10°C, or the numerical value within a range consisting of any two values thereof. Further preferably, the starting reduction temperature of the hydrogen leakage detecting material on hydrogen gas is less than or equal to 50°C, more preferably less than or equal to 43°C.

In some embodiments of the present disclosure, the reflectivity of said hexagonal-phase tungsten trioxide to visible light within a wavelength range of 400-750nm as measured by a spectrometer is larger than or equal to 40%, preferably larger than or equal to 45%, when using a standard that the reflectivity of barium sulfate is equal to 100%. Preferably, when using a standard that the reflectivity of barium sulfate is equal to 100%, the reflectivity of said hexagonal-phase tungsten trioxide to visible light within a wavelength range of 400-750nm as measured by a spectrometer may be any value of 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, or the numerical value within a range consisting of any two values thereof.

In some embodiments of the present disclosure, the hexagonal-phase tungsten trioxide has a reflectivity to blue light within a wavelength range of 400-500nm as measured by a spectrometer being larger than or equal to 45%, and a reflectivity to red light within a wavelength range of 600-750nm being larger than or equal to 49%, when using a standard that the reflectivity of barium sulfate is equal to 100%. Preferably, when using a standard that the reflectivity of barium sulfate is equal to 100%, the reflectivity of the hexagonal-phase tungsten trioxide to blue light within a wavelength range of 400-500nm as measured by a spectrometer may be any value of 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, or the numerical value within a range consisting of any two values thereof; the reflectivity of the hexagonal-phase tungsten trioxide to red light within a wavelength range of 600-750nm may be any value of 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, or the numerical value within a range consisting of any two values thereof.

In the present disclosure, the H₂ concentration refers to the percentage of H₂ in the total volume of H₂ and Ar in the H₂/Ar mixture at room temperature. The hydrogen gas with different concentrations refers to the H₂/Ar mixture with different H₂ concentrations, and vice versa.

In the present disclosure, the term "room temperature" refers to "25°C".

In some embodiments of the present disclosure, a test sample is obtained after contacting the hydrogen leakage detecting material with hydrogen gas having a concentration of 10vol.% for 60s, wherein the conditions of introducing hydrogen gas comprise: a flow rate of 100mL/min at room temperature. In some embodiments of the present disclosure, when using a standard that the reflectivity of barium sulfate is equal to 100%, the reflectivity of the test sample to visible light within a wavelength range of 400-750nm as measured by a spectrometer is less than or equal to 30%. Preferably, when using a standard that the reflectivity of barium sulfate is equal to 100%, the reflectivity of the test sample to visible light within a wavelength range of 400-750nm as measured by a spectrometer may be any value of 0%, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, or the numerical value within a range consisting of any two values thereof.

In some embodiments of the present disclosure, when using a standard that the reflectivity of barium sulfate is equal to 100%, the reflectivity of the test sample to blue light within a wavelength range of 400-500nm as measured by a spectrometer is less than or equal to 30%, the reflectivity of the test sample to red light within a wavelength range of 600-750nm as measured by a spectrometer is less than or equal to 20%. Preferably, when using a standard that the reflectivity of barium sulfate is equal to 100%, the reflectivity of the test sample to blue light within a wavelength range of 400-500nm as measured by a spectrometer may be any value of 0%, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, or the numerical value within a range consisting of any two values thereof; the reflectivity of the test sample to red light within a wavelength range of 600-750nm as measured by a spectrometer may be any value of 0%, 1%, 2%, 3%, 4%, 5%, 6%, 7%, 8%, 9%, 10%, 11%, 12%, 13%, 14%, 15%, 16%, 17%, 18%, 19%, 20%, or the numerical value within a range consisting of any two values thereof. In addition, the difference between the lowest value of the reflectivity of the test sample to blue light within a wavelength range of 400-500nm and the highest value of the reflectivity of the test sample to red light within a wavelength range of 600-750nm is larger than or equal to 5%.

In the present disclosure, it is possible to obtain a test sample and determine the reflectivity of the test sample by using a geochromism testing device and an in-situ testing system (CN202110955330.6 describes the device and system, the content of which is entirely incorporated herein by reference), the testing method is as follows: placing a sample cell having a diameter of 2 cm and a thickness of 1 cm in an enclosed reaction chamber having a diameter of 10 cm diameter and a thickness of 5 cm), planishing the hydrogen leakage detecting material in the sample cell, contacting the test sample with hydrogen gas having a concentration of 10vol.% introduced at room temperature for 60s, and performing the real-time testing on the color change process by using a spectrometer to obtain reflectivity data of the test sample.

In some embodiments of the present disclosure, the reflectivity change difference of the test sample to visible light within the wavelength range of 400-750nm is larger than or equal to 20%. Preferably, when using a standard that the reflectivity of barium sulfate is equal to 100%, the reflectivity change difference of the test sample to visible light within the wavelength range of 400-750nm as measured by a spectrometer may be any value of 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, or the numerical value within a range consisting of any two values thereof.

In some embodiments of the present disclosure, the test sample has a reflectivity change difference to blue light within the wavelength range of 400-500nm being larger than or equal to 22%, and a reflectivity change difference to red light within the wavelength range of 600-750nm being larger than or equal to 30%. Preferably, when using a standard that the reflectivity of barium sulfate is equal to 100%, the reflectivity change difference of the test sample to blue light within the wavelength range of 400-500nm as measured by a spectrometer may be any value of 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, or the numerical value within a range consisting of any two values thereof; the reflectivity change difference of the test sample to red light within the wavelength range of 600-750nm as measured by a spectrometer may be any value of 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, or the numerical value within a range consisting of any two values thereof.

In the present disclosure, the reflectivity change difference refers to a difference between the reflectivity of hydrogen leakage detecting material before introducing hydrogen gas and the reflectivity of hydrogen leakage detecting material after introducing hydrogen gas. Specifically, the difference between the reflectivity of hydrogen leakage detecting material before introducing hydrogen gas and the reflectivity of hydrogen leakage detecting material after introducing hydrogen gas refers to the difference of reflectivity of the hydrogen leakage detecting material to the light having the same wavelength before and after introducing hydrogen gas. It shall be noted that the lowest value of the reflectivity change difference of the hydrogen leakage detecting material to the light within a wavelength range refers to the lowest value of the reflectivity change difference of the hydrogen leakage detecting material to the light within a wavelength range before and after introducing hydrogen gas. For example, the reflectivity change difference of the hydrogen leakage detecting material to blue light within the wavelength range of 400-500nm before introducing hydrogen gas is between 50% and 60%, wherein the reflectivity to the light at the wavelength of 450nm is 55%; the reflectivity change difference of the hydrogen leakage detecting material to blue light within the wavelength range of 400-500nm after introducing hydrogen gas is between 20% and 30%, wherein the reflectivity to the light at the wavelength of 450nm is 30%, thus the reflectivity change difference of the hydrogen leakage detecting material to the light at a wavelength of 450nm is 25%; if the reflectivity change difference of the material to the light at a wavelength of 450nm is lower than the reflectivity change difference of the material to the light at other wavelength within the wavelength range of 400-500nm, the reflectivity change difference of the hydrogen leakage detecting material to blue light within the wavelength range of 400-500nm is larger than or equal to 25%. In some embodiments of the present disclosure, the hydrogen leakage detecting material has a color-changing response time of less than or equal to 2s for pure hydrogen gas, a color-changing response time of less than or equal to 5s for hydrogen gas having a concentration of 10vol.%, a color-changing response time of less than or equal to 10s for hydrogen gas having a concentration of 4vol.%, a color-changing response time of less than or equal to 13s for hydrogen gas having a concentration of 2 vol.%, a color-changing response time of less than or equal to 15s for hydrogen gas having a concentration of 1 vol.%, a color-changing response time of less than or equal to 25s for hydrogen gas having a concentration of 0.5vol.%, and a color-changing response time of less than or equal to 50s for hydrogen gas having a concentration of 0.1 vol.%. Wherein the detection conditions of the color-changing response time are as follows: the hydrogen gas flow rate is 200mL/min at room temperature. Preferably, the color-changing response time of the hydrogen leakage detecting material for pure hydrogen gas may be any value of 0.5s, 1s, 1.5s, 2s, or the numerical value within a range consisting of any two values thereof; the color-changing response time of the hydrogen leakage detecting material for hydrogen having a concentration of 10vol.% may be any value of 0.5s, 1s, 1.5s, 2s, 2.5s, 3s, 3.5s, 4s, 4.5s, 5s, or the numerical value within a range consisting of any two values thereof; the color-changing response time of the hydrogen leakage detecting material for hydrogen having a concentration of 4vol.% may be any value of 0.5s, 1s, 1.5s, 2s, 2.5s, 3s, 3.5s, 4s, 4.5s, 5s, 5.5s, 6s, 6.5s, 7s, 7.5s, 8s, 8.5s, 9s, 9.5s, 10s, or the numerical value within a range consisting of any two values thereof; the color-changing response time of the hydrogen leakage detecting material for hydrogen having a concentration of 2vol. % may be any value of 0.5s, 1s, 1.5s, 2s, 2.5s, 3s, 3.5s, 4s, 4.5s, 5s, 5.5s, 6s, 6.5s, 7s, 7.5s, 8s, 8.5s, 9s, 9.5s, 10s, 10.5s, 11s, 11.5s, 12s, 12.5s, 13s, or the numerical value within a range consisting of any two values thereof; the color-changing response time of the hydrogen leakage detecting material for hydrogen having a concentration of 1vol.% may be any value of 0.5s, 1s, 1.5s, 2s, 2.5s, 3s, 3.5s, 4s, 4.5s, 5s, 5.5s, 6s, 6.5s, 7s, 7.5s, 8s, 8.5s, 9s, 9.5s, 10s, 10.5s, 11s, 11.5s, 12s, 12.5s, 13s, 13.5s, 14s, 14.5s, 15s, or the numerical value within a range consisting of any two values thereof; the color-changing response time of the hydrogen leakage detecting material for hydrogen having a concentration of 0.5vol.% may be any value of 0.5s, 1s, 1.5s, 2s, 2.5s, 3s, 3.5s, 4s, 4.5s, 5s, 5.5s, 6s, 6.5s, 7s, 7.5s, 8s, 8.5s, 9s, 9.5s, 10s, 10.5s, 11s, 11.5s, 12s, 12.5s, 13s, 13.5s, 14s, 14.5s, 15s, 15.5s, 16s, 16.5s, 17s, 17.5s, 18s, 18.5s, 19s, 19.5s, 20s, 20.5s, 21s, 21.5s, 22s, 22.5s, 23s, 23.5s, 24s, 24.5s, 25s, or the numerical value within a range consisting of any two values thereof; the color-changing response time of the hydrogen leakage detecting material for hydrogen having a concentration of 0.1 vol. % may be any value of 0.5s, 1s, 1.5s, 2s, 2.5s, 3s, 3.5s, 4s, 4.5s, 5s, 5.5s, 6s, 6.5s, 7s, 7.5s, 8s, 8.5s, 9s, 9.5s, 10s, 10.5s, 11s, 11.5s, 12s, 12.5s, 13s, 13.5s, 14s, 14.5s, 15s, 15.5s, 16s, 16.5s, 17s, 17.5s, 18s, 18.5s, 19s, 19.5s, 20s, 20.5s, 21s, 21.5s, 22s, 22.5s, 23s, 23.5s, 24s, 24.5s, 25s, 26s, 27s, 28s, 29s, 30s, 31s, 32s, 33s, 34s, 35s, 36s, 37s, 38s, 39s, 40s, 41s, 42s, 43s, 44s, 45s, 46s, 47s, 48s, 49s, 50s, or the numerical value within a range consisting of any two values thereof.

In the present disclosure, the term "color-changing response time" is defined as the time required for that the color of the hydrogen leakage detecting material after introducing hydrogen gas exhibits the significant and visible change, and the reflectivity change difference to the light at the wavelength of 700nm reaches 10%.

In the present disclosure, the color-changing response time of the hydrogen leakage detecting material can be determined by using a gasochromic testing device and an in-situ testing system (CN202110955330.6 describes the device and system, the content of which is entirely incorporated herein by reference), the testing method is as follows: placing a sample cell having a diameter of 2 cm and a thickness of 1 cm in an air-tight gas chamber (having a diameter of 10 cm diameter and a thickness of 5 cm), planishing the hydrogen leakage detecting material in the sample cell, then introducing hydrogen gas under the specific conditions, and performing the real-time testing on the color change process by using a spectrometer; that is, performing the real-time recording of the reflection spectrum data of the hydrogen leakage detecting material before and during the process of introducing hydrogen gas, and accordingly obtaining the color-changing response time, i.e., the time required for that the reflectivity change difference of the hydrogen leakage detecting material to the light at the wavelength of 700nm reaches 10%.

In some embodiments of the present disclosure, the hydrogen leakage detecting material has a color-changing response time of less than or equal to 2.5s at room temperature for hydrogen gas at a flow rate of 500mL/min, a color-changing response time of less than or equal to 3s for hydrogen gas at a flow rate of 400mL/min, a color-changing response time of less than or equal to 4s for hydrogen gas at a flow rate of 300mL/min, a color-changing response time of less than or equal to 5s for hydrogen gas at a flow rate of 200mL/min, a color-changing response of time less than or equal to 8s for hydrogen gas at a flow rate of 100mL/min, a color-changing response time of less than or equal to 10s for hydrogen gas at a flow rate of 50mL/min, a color-changing response time of less than or equal to 20s for hydrogen gas at a flow rate of 20mL/min. wherein the testing conditions of the color-changing response time are as follows: room temperature, the hydrogen gas has a concentration of 10vol.%. Preferably, the color change response time of the hydrogen leakage detecting material at room temperature for hydrogen gas at a flow rate of 500mL/min may be any value of 0.5s, 1s, 1.5s, 2s, or the numerical value within a range consisting of any two values thereof; the color change response time of the hydrogen leakage detecting material for hydrogen gas at a flow rate of 400mL/min may be any value of 0.5s, 1s, 1.5s, 2s, 2.5s, 3s, or the numerical value within a range consisting of any two values thereof; the color change response time of the hydrogen leakage detecting material for hydrogen gas at a flow rate of 300mL/min may be any value of 0.5s, 1s, 1.5s, 2s, 2.5s, 3s, 35s, 4s, or the numerical value within a range consisting of any two values thereof; the color change response time of the hydrogen leakage detecting material for hydrogen gas at a flow rate of 200mL/min may be any value of 0.5s, 1s, 1.5s, 2s, 2.5s, 3s, 35s, 4s, 4.5s, 5s, or the numerical value within a range consisting of any two values thereof; the color change response time of the hydrogen leakage detecting material for hydrogen gas at a flow rate of 100mL/min may be any value of 0.5s, 1s, 1.5s, 2s, 2.5s, 3s, 35s, 4s, 4.5s, 5s, 5.5s, 6s, 6.5s, 7s, 7.5s, 8s, or the numerical value within a range consisting of any two values thereof; the color change response time of the hydrogen leakage detecting material for hydrogen gas at a flow rate of 50mL/min may be any value of 0.5s, 1s, 1.5s, 2s, 2.5s, 3s, 35s, 4s, 4.5s, 5s, 5.5s, 6s, 6.5s, 7s, 7.5s, 8s, 8.5s, 9s, 9.5s, 10s, or the numerical value within a range consisting of any two values thereof; the color change response time of the hydrogen leakage detecting material for hydrogen gas at a flow rate of 20mL/min may be any value of 0.5s, 1s, 1.5s, 2s, 2.5s, 3s, 35s, 4s, 4.5s, 5s, 5.5s, 6s, 6.5s, 7s, 7.5s, 8s, 8.5s, 9s, 9.5s, 10s, 10.5s, 11s, 11.5s, 12s, 12.5s, 13s, 13.5s, 14s, 14.5s, 15s, 15.5s, 16s, 16.5s, 17s, 17.5s, 18s, 18.5s, 19s, 19.5s, 20s, or the numerical value within a range consisting of any two values thereof.

In some embodiments of the present disclosure, the hydrogen leakage detecting material has a color-changing response time of less than or equal to 27s for hydrogen gas having a concentration of 10vol.% at the temperature of -25°C, a color-changing response time of less than or equal to 15s for hydrogen gas at the temperature of -15°C, a color-changing response time of less than or equal to 13s for hydrogen gas at the temperature of -5°C, a color-changing response time of less than or equal to 8s for hydrogen gas at the temperature of 5°C, a color-changing response time of less than or equal to 6.5s for hydrogen at the temperature of 15°C, a color-changing response time of less than or equal to 5s for hydrogen gas at the temperature of 25°C, a color-changing response time of less than or equal to 4s for hydrogen gas at the temperature of 35°C, a color-changing response time of less than or equal to 3s for hydrogen gas at the temperature of 45°C, wherein the testing conditions of the color-changing response time are as follows: a flow rate of hydrogen is 200mL/min, a concentration of hydrogen gas is 10vol.%. Preferably, the color-changing response time of the hydrogen leakage detecting material for hydrogen gas having a concentration of 10vol.% at the temperature of -25°C may be any value of 0.5s, 1s, 1.5s, 2s, 2.5s, 3s, 3.5s, 4s, 4.5s, 5s, 5.5s, 6s, 6.5s, 7s, 7.5s, 8s, 8.5s, 9s, 9.5s, 10s, 10.5s, 11s, 11.5s, 12s, 12.5s, 13s, 13.5s, 14s, 14.5s, 15s, 15.5s, 16s, 16.5s, 17s, 17.5s, 18s, 18.5s, 19s, 19.5s, 20s, 20.5s, 21s, 21.5s, 22s, 22.5s, 23s, 23.5s, 24s, 24.5s, 25s, 25.5s, 26s, 26.5s, 27s, or the numerical value within a range consisting of any two values thereof; the color-changing response time of the hydrogen leakage detecting material for hydrogen gas at the temperature of -15°C may be any value of 0.5s, 1s, 1.5s, 2s, 2.5s, 3s, 3.5s, 4s, 4.5s, 5s, 5.5s, 6s, 6.5s, 7s, 7.5s, 8s, 8.5s, 9s, 9.5s, 10s, 10.5s, 11s, 11.5s, 12s, 12.5s, 13s, 13.5s, 14s, 14.5s, 15s, or the numerical value within a range consisting of any two values thereof; the numerical value within a range consisting of any two values thereof; the color-changing response time of the hydrogen leakage detecting material for hydrogen gas at the temperature of -5°C may be any value of 0.5s, 1s, 1.5s, 2s, 2.5s, 3s, 3.5s, 4s, 4.5s, 5s, 5.5s, 6s, 6.5s, 7s, 7.5s, 8s, 8.5s, 9s, 9.5s, 10s, 10.5s, 11s, 11.5s, 12s, 12.5s, 13s, or the numerical value within a range consisting of any two values thereof; the color-changing response time of the hydrogen leakage detecting material for hydrogen gas at the temperature of 5°C may be any value of 0.5s, 1s, 1.5s, 2s, 2.5s, 3s, 3.5s, 4s, 4.5s, 5s, 5.5s, 6s, 6.5s, 7s, 7.5s, 8s, or the numerical value within a range consisting of any two values thereof; the color-changing response time of the hydrogen leakage detecting material for hydrogen gas at the temperature of 15°C may be any value of 0.5s, 1s, 1.5s, 2s, 2.5s, 3s, 3.5s, 4s, 4.5s, 5s, 5.5s, 6s, 6.5s, or the numerical value within a range consisting of any two values thereof; the color-changing response time of the hydrogen leakage detecting material for hydrogen gas at the temperature of 25°C may be any value of 0.5s, 1s, 1.5s, 2s, 2.5s, 3s, 3.5s, 4s, 4.5s, 5s, or the numerical value within a range consisting of any two values thereof; the color-changing response time of the hydrogen leakage detecting material for hydrogen gas at the temperature of 35°C may be any value of 0.5s, 1s, 1.5s, 2s, 2.5s, 3s, 3.5s, 4s, or the numerical value within a range consisting of any two values thereof; the color-changing response time of the hydrogen leakage detecting material for hydrogen gas at the temperature of 45°C may be any value of 0.5s, 1s, 1.5s, 2s, 2.5s, 3s, or the numerical value within a range consisting of any two values thereof.

A second aspect of the present disclosure provides a method for preparing the aforementioned hydrogen leakage detecting material comprising the following steps:
(i) carrying out a first mixing for a noble metal precursor, tungsten trioxide and deionized water to obtain a solid-liquid mixture;
(ii) subjecting an organic liquid phase reduction reagent and the solid-liquid mixture to a second mixing to obtain a reaction mixture;
(iii) subjecting the reaction mixture to an organic liquid phase reduction;
wherein the tungsten trioxide is hexagonal-phase tungsten trioxide, and has a microstructure of a nanorod bundle formed by stacking multiple nanorods.

In some embodiments of the present disclosure, the addition amount of the noble metal precursor is 0.2-5 wt.%, preferably 0.5-2wt.%, relative to the addition amount of the tungsten trioxide.

The inventors have found in the researches when the addition amount of the noble metal precursor exceeds 5wt.% of the addition amount of tungsten trioxide, the noble metal dispersity of the produced hydrogen leaking material decreases. Further, when the addition amount of the noble metal precursor is 0.5-2wt.% relative to the addition amount of tungsten trioxide, the noble metal dispersity of the produced hydrogen leakage detecting material is high and exceeding 70%, and the hydrogen sensitive color change performance is desirable.

Preferably, the addition amount of the noble metal precursor calculated in terms of noble metal may be any value of 0.5 wt.%, 0.6 wt.%, 0.7 wt.%, 0.8 wt.%, 0.9 wt.%, 1 wt.%, 1.5 wt.%, 2wt.%, or the numerical value within a range consisting of any two values thereof, relative to the addition amount of tungsten trioxide.

The addition amount of deionized water is not particularly limited in the present disclosure, as long as the solid content of the solid-liquid mixture is within a range of 5-70 wt.%.

In some embodiments of the present disclosure, the addition mass of the organic liquid phase reducing reagent is 1.5-12 times of the addition mass of the tungsten trioxide.

Preferably, the addition mass of the organic liquid phase reducing reagent may be any value of 1.5 times, 2 times, 2.5 times, 3 times, 3.5 times, 4 times, 4.5 times, 5 times, 7 times, 10 times, 12 times, or the numerical value within a range consisting of any two values thereof, relative to the addition mass of the tungsten trioxide.

In some embodiments of the present disclosure, the noble metal precursor is a noble metal soluble salt, preferably a soluble salt of at least one selected from the group consisting of platinum, palladium, rhodium and gold.

In some embodiments of the present disclosure, the noble metal soluble salt is a soluble salt of platinum, for example, it may be at least one selected from the group consisting of potassium hexachloroplatinate, platinum(II) nitrate, chloroplatinic acid, sodium tetrachloroplatinate(II), potassium tetranitroplatinate(II), ammonium hexachloroplatinate(IV) and potassium tetrachloroplatinate(II).

In some embodiments of the present disclosure, the organic liquid phase reducing reagent is an organic alcohol and/or a first organic acid, wherein the first organic acid is at least one selected from the group consisting of ascorbic acid, oxalic acid and citric acid, and the organic alcohol is at least one selected from the group consisting of ethylene glycol, propylene glycol and butylene glycol.

An increase in the dispersion of the noble metal nanoparticles on tungsten trioxide in the hydrogen leakage detecting material is conducive to reducing the starting reduction temperature of the hydrogen leakage detecting material on hydrogen gas. The inventors have discovered in researches when the organic liquid phase reducing reagent is a composition of an organic alcohol and a first organic acid, the resulting hydrogen leakage detecting material has a higher dispersity of the noble metal and a further reduced staring reduction temperature on hydrogen gas. Therefore, it is preferable that the organic liquid phase reducing reagent is a composition of an organic alcohol and a first organic acid, and the mass ratio of the organic alcohol to the first organic acid is 1: 0.1-5, preferably 1:0.1-0.3.

The present disclosure does not impose requirements on the mode and time of the first mixing, as long as the noble metal precursor, tungsten trioxide and deionized water are completely mixed. Preferably, the second mixing mode is stirring for a time period of 10-30min.

The present disclosure does not impose requirements on the mode and time of the second mixing, as long as the organic liquid-phase agent is thoroughly blended with the solid-liquid mixture. Preferably, the second mixing is stirring for a time period of 10-30min.

In some embodiments of the present disclosure, the conditions of the organic liquid phase reduction comprise: the reduction temperature within a range of 105-180°C, preferably within a range of 115-160°C, and the reduction time within a range of 0.5-8h, preferably within a range of 1.5-6 h.

The inventors have found in the present disclosure that if the reduction temperature is lower than 105°C, the reduction reaction rate is too low or even the reduction cannot occur, thus the deposition of metal nanoparticles in tungsten trioxide cannot be realized; if the reduction temperature is higher than 180 °C , the metal reduction speed in the metal soluble salt is too high, so that the metal nanoparticles are agglomerated on the surface of tungsten trioxide, the metal nanoparticles of the prepared hydrogen leakage detecting material have large particle size and poor dispersity, thus the detection sensitivity is decreased.

In some embodiments of the present disclosure, the step (iii) further comprises centrifugation treatment, washing treatment and drying treatment after the organic liquid phase reduction. In some embodiments of the present disclosure, the washing treatment refers to washing with deionized water and absolute ethanol. The present disclosure does not impose requirement on the number of washing operation, as long as the pH of an eluate is within a range of 6.5-7.5.

Preferably, the conditions of drying treatment are as follows: the temperature is within a range of 60-110°C, and the time is 4-36h. Further preferably, the conditions of drying treatment are as follows: the temperature is within a range of 80-105°C, and the time is 8-24h.

In some embodiments of the present disclosure, the method of preparing tungsten trioxide comprises the following steps:
(1) blending an inorganic acid, an organic additive, a structure-directing agent and a tungsten salt solution to obtain a mixed solution;
(2) subjecting the mixed solution to a hydrothermal reaction in a hydrothermal reaction kettle.

In some embodiments of the present disclosure, the tungsten salt contained in the tungsten salt solution is a tungsten salt containing no ammonium radical.

In some embodiments of the present disclosure, the molar ratio of the mole number of the tungsten salt relative to the total mole number of the organic additive and the inorganic acid is within a range of 0.1-4:1, preferably 0.2-3:1.

Preferably, the molar ratio of the mole number of the tungsten salt relative to the total mole number of the organic additive and the inorganic acid may be any value of 0.2:1, 0.3:1, 0.4:1, 0.5:1, 0.6:1, 0.7:1, 0.8:1, 0.9:1, 1:1, 2:1, 3:1, or the numerical value within a range consisting of any two values thereof.

The present disclosure has no requirements for the solvent and volume of the tungsten salt solution, as long as the solvent can dissolve the tungsten salt. Preferably, the solvent of the tungsten salt solution is deionized water.

In some embodiments of the present disclosure, the mole number of the organic additive is 1-95% of the total mole number of the organic additive and the inorganic acid.

Preferably, the mole number of the organic additive relative to the total mole number of the organic additive and the inorganic acid may be any value of 1%, 2%, 3%, 4%, 5%, 6%, 7%, 8%, 9%, 10%, 20%, 30%, 40%, 50%, 60%, 70%, 80%, 85%, 90%, 95%, or the numerical value within a range consisting of any two values thereof.

In the present disclosure, the term "total mole number of the organic additive and the inorganic acid" refers to the sum of the mole number of the organic additive and the mole number of the inorganic acid, and the term "mole number of the organic additive" refers to the mole number of said organic additive.

In some embodiments of the present disclosure, the mass ratio of the tungsten salt to the structure-directing agent is within a range of 0.1-4:1, preferably within a range of 0.2-3:1.

Preferably, the mass ratio of the tungsten salt to the structure-directing agent may be any value of 0.2:1, 0.3:1, 0.4:1, 0.5:1, 0.6:1, 0.7:1, 0.8:1, 0.9:1, 1:1, 1.5:1, 2:1, 2.1:1, 2.3:1, 2.5:1, 2.7:1, 2.9:1, 3:1, or the numerical value within a range consisting of any two values thereof.

In some embodiments of the present disclosure, the inorganic acid is at least one selected from the group consisting of hydrochloric acid, nitric acid and sulfuric acid.

In some embodiments of the present disclosure, the organic additive is an organic acid salt and/or a second organic acid. Preferably, the organic acid salt is at least one selected from the group consisting of sodium hexadecyl benzene sulfonate and sodium hexadecyl sulfonate, and the second organic acid is at least one selected from the group consisting of citric acid and oxalic acid.

In the present disclosure, the structure-directing agent is an inorganic structure-directing agent, preferably at least one selected from the group consisting of chloride, sulfate and nitrate of group IA metal elements, and more preferably, at least one selected from the group consisting of lithium chloride, sodium chloride, potassium chloride, lithium sulfate, sodium sulfate, lithium bisulfate, sodium bisulfate, potassium bisulfate, lithium nitrate, sodium nitrate and potassium nitrate.

The inventors have unexpectedly found in researches that when a tungsten salt containing an ammonium radical (e.g., ammonium metatungstate) is used, the prepared tungsten trioxide does not have the regular nanorod bundle structure and is dark in color. Therefore, the tungsten salt in the present disclosure is a tungsten salt without ammonium radicals, preferably at least one selected from the group consisting of sodium tungstate, phosphotungstic acid, sodium phosphotungstate, lithium tungstate, and potassium tungstate.

In some embodiments of the present disclosure, the hydrothermal reaction is carried out at a temperature of 140-240°C, preferably a temperature of 150-220°C for a time period of 1-48 h, preferably 4-30 h.

In some embodiments of the present disclosure, the blending process in step (1) refers to that the inorganic acid is added to the tungsten salt solution, the organic additive and the structure-directing agent are subsequently added.

In some embodiments of the present disclosure, the step (2) further comprises a centrifugation treatment, a washing treatment and a drying treatment after the hydrothermal reaction.

Preferably, the washing treatment refers to washing with deionized water and absolute ethyl alcohol.

Preferably, the conditions of drying treatment are as follows: the temperature is within a range of 60-120°C, and the time period is 2-26 h.

A third aspect of the present disclosure provides a use of the hydrogen leakage detecting material in the hydrogen-containing reducing gas leakage detection product.

Some embodiments of the present disclosure provide a use of the hydrogen leakage detecting material in the hydrogen leakage detection product, the hydrogen leakage detection product may include, but is not limited to, a hydrogen leakage detection sensor, a hydrogen leakage detection tape, a hydrogen leakage detection coating, a hydrogen leakage detection component, a hydrogen leakage detection film, and a hydrogen leakage detection ink.

Besides hydrogen gas, the hydrogen leakage detection material disclosed by the present disclosure can also be applied to the hydrogen-containing reducing gas leakage detection product for other gases such as hydrogen sulfide, ammonia gas, ethyl alcohol.

The present disclosure will be described in detail below with reference to examples, but the present disclosure is not limited to the following examples.

Unless otherwise specified in the present disclosure, the experimental methods used in the following examples and comparative examples were all conventional methods, the raw materials, reagents and the like used in the following examples and comparative examples were commercially available. The room temperature denoted "25°C".

In the following examples and comparative examples:

The dilute hydrochloric acid, dilute sulfuric acid and dilute nitric acid were respectively obtained by diluting concentrated hydrochloric acid, concentrated sulfuric acid and concentrated nitric acid with deionized water. Wherein the concentrated hydrochloric acid is purchased from the Sinopharm Chemical Reagent Co., Ltd., and the concentration was 37%; the concentrated sulfuric acid was purchased from the Sinopharm Chemical Reagent Co., Ltd., and the concentration was 98%; the concentrated nitric acid was purchased from the Sinopharm Chemical Reagent Co., Ltd., the concentration was 65%.

The "platinum species" denoted the species containing platinum element, such as a simple substance, an oxide.

The H₂ concentration referred to the percentage of H₂ in the total volume of H₂ and Ar in the H₂/Ar mixture at room temperature. The hydrogen gas with different concentrations referred to the H₂/Ar mixture with different H₂ concentrations, and vice versa.

The XRD diffraction spectrogram was obtained by testing with an X-ray diffractometer, which was manufactured by Panalytical B.V. in the Netherlands with a model name X' Pert Pro MPD.

The SEM photographs were obtained by Scanning Electron Microscope (SEM) commercially available from the Hitachi Corporation in Japan with the model name S-4800.

The TEM and HRTEM photographs were obtained by a high-power transmission electron microscope purchased from the JEOL Corporation in Japan, with the model name JEM-2100, and element distribution maps were obtained by the Energy Dispersive Spectrometer (EDS) attached to the high-power transmission electron microscope.

The dispersity was determined by the quantitative hydrogen pulse chemical reaction by using a high-pressure chemical adsorption instrument purchased from the Micrometritics Instrument Corporation in USA with the model name HP 2950. In the testing process, the hydrogen leakage detecting material was firstly reduced in H₂/Ar mixed gas at a flow rate of 50mL/min to the maximum temperature of 400°C, then cooled to the room temperature under the inert atmosphere, a certain amount (0.5 mL) of H₂/Ar mixed gas was finally and continuously introduced in a pulse manner, the hydrogen consumption was calculated with the algorithm attached in the high-pressure chemical adsorption instrument to obtain the dispersity. Wherein the concentration of hydrogen gas in the H₂/Ar mixed gas was 10 vol.%. The inert atmosphere was Ar gas.

TPR analysis was used to illustrate the starting reduction temperature of the hydrogen leakage detecting material on hydrogen gas, the temperature was obtained by the chemical adsorption instrument purchased from the Micrometritics Instrument Corporation in the USA with a model name 2950 HP. Wherein the starting reduction temperature on hydrogen gas was the starting temperature when the first significant hydrogen consumption peak occurred.

The content of noble metal nanoparticles in the hydrogen leakage detecting material was detected by an X-ray fluorescence analysis instrument, which was purchased from the Bruker Corporation in Germany with a model name S8.

The reflection spectrum and the reflectivity were collected by a spectrometer, which was purchased from the Ocean Optics Corporation in USA with the model name QE Pro, and the detection conditions were based on the standard: the reflectivity of barium sulfate was equal to 100%.

The following detections were performed using a geochromism testing device and an in-situ testing system (CN202110955330.6 described the device and system, the content of which was entirely incorporated herein by reference), the detection method was as follows: a sample cell having a diameter of 2 cm and a thickness of 1 cm was placed in an air-tight gas chamber (having a diameter of 10 cm diameter and a thickness of 5 cm), the hydrogen leakage detecting material to be tested was placed in the sample cell, and planished, the hydrogen gas was then introduced under the specific conditions according to the experimental requirements, the real-time testing on the color change process was performed by using a spectrometer and a high speed camera; the real-time recording of the reflection spectrum and the color change data of the hydrogen leakage detecting material before and during the process of introducing hydrogen gas, and the corresponding analysis was performed:
1. The hydrogen gas under the specific conditions was introduced for a specific time, the reflection spectrum and the color of the hydrogen leakage detecting material before and after introducing hydrogen gas were compared and analyzed, so as to reflect the reflectivity change of the hydrogen leakage detecting material before and after introducing hydrogen gas.
2. The hydrogen leakage detection referred to the color-changing response time of the hydrogen leakage detecting material to hydrogen gas with different H₂ concentrations, different temperatures and different flow rates, it was used for reflecting the detection sensitivity of the hydrogen leakage detecting material. Wherein the color-changing response time referred to the time required for that the color of hydrogen leakage detecting material after introducing hydrogen gas exhibited the significant and visible change, and the reflectivity change difference of the hydrogen leakage detecting material to the light at the wavelength of 700nm reached 10%.

The detection effect of the hydrogen leakage detecting material was represented by the color change before and after introducing hydrogen gas and the reflectivity change difference of the hydrogen leakage detecting material to visible light within the wavelength range of 400-750nm; the larger were the difference in color change and the reflectivity change, the better was the effect of detecting hydrogen leakage of the hydrogen leakage detecting material.

### Example 1

(1) The certain amounts of sodium tungstate, oxalic acid and sodium sulfate were weighted, the diluted hydrochloric acid with a concentration of 2.5M was measured and taken, wherein the specific weighed and measured amounts were shown in Table 1.
(2) Sodium tungstate was dissolved in 50 mL of deionized water to prepare a sodium tungstate solution, dilute hydrochloric acid was added into the sodium tungstate solution and stirred for 25 min, wherein the adding speed of the dilute hydrochloric acid was 3 mL/min. After the addition of dilute hydrochloric acid was complete, oxalic acid and sodium sulfate were added into the mixed solution, and continuously stirred for 30 min.
(3) The stirred solution was transferred to a hydrothermal reaction kettle with polytetrafluoroethylene as an inner lining and stainless steel as an outer protective sleeve, the hydrothermal reaction kettle was screwed tightly, the hydrothermal reaction was then performed at 160°C for 12 h, and naturally cooled to room temperature.
(4) The reaction product was subjected to centrifugal separation, then washed with deionized water and absolute ethyl alcohol for multiple times until the pH of an eluate was within a range of 6.5-7, the reaction product was subjected to drying in a baking oven at 100°C for 12h to obtain tungsten trioxide A1.

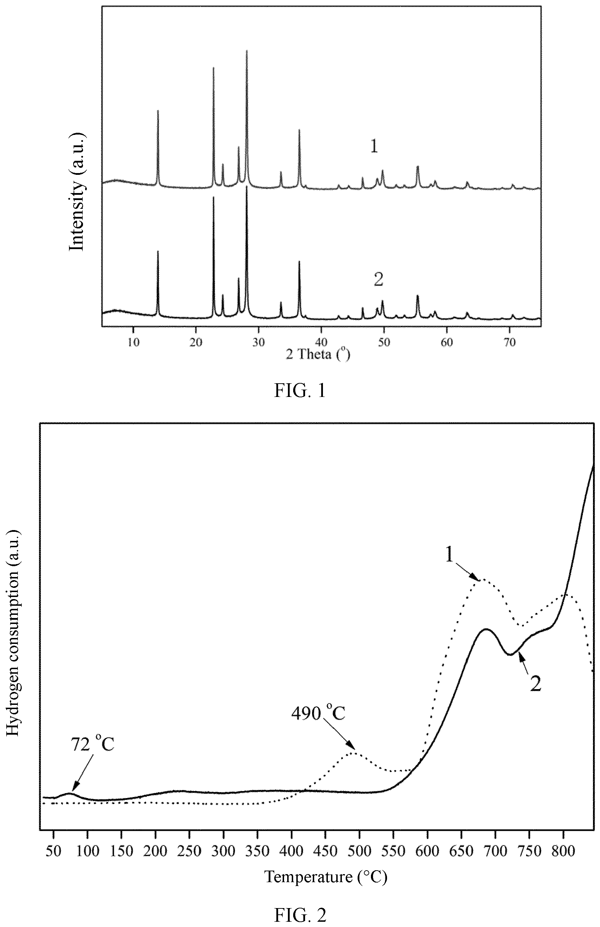

The tungsten trioxide A1 obtained in Example 1 was analyzed with respect to crystalline phase, micro-morphology and reflection spectrogram, the relevant analysis results were shown in FIG. 1, FIGS. 3-5 and Table 2.

FIG. 1 illustrated an XRD analysis of tungsten trioxide A1, which was compared with the standard card (JCPDS 33-1387), the comparison result showed that the crystalline phase of tungsten trioxide A1 was hexagonal-phase. In addition, a diffraction peak of 2θ at around 28° was attributed to the (002) crystal plane, a diffraction peak of 2θ at around 22° was attributed to the (100) crystal plane, and a diffraction peak of 2θ at around 14° was attributed to the (001) crystal plane. The relative intensities of the three characteristic diffraction peaks satisfied the following conditions: I₍₀₀₂₎=1.07×I₍₁₀₀₎=1.87×I₍₀₀₁₎.

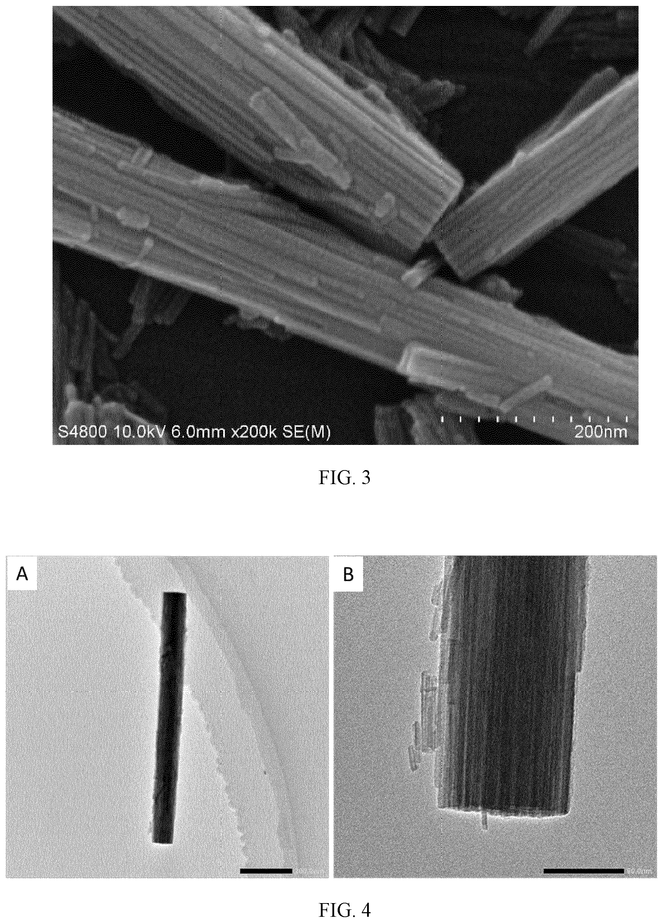

FIG. 3, FIG. 4 and FIG. 5 illustrated the SEM analysis, the TEM analysis, and the HRTEM analysis of tungsten trioxide A1, respectively. As shown in FIG. 3, the micro-morphology of the tungsten trioxide A1 was a microstructure of nanorod bundle formed by a plurality of nanorods with a diameter about 8nm adhered to each other and stacked together. As illustrated in FIG. 4, the micro-morphology of tungsten trioxide was a nanorod bundle formed by stacking a plurality of nanorods, the nanorod bundle had a length about 900nm and a diameter about 80nm, the single nanorod had a diameter about 8 nm, a length-diameter ratio of the nanorod bundle was 11.25.

As shown in FIG. 8 and FIG. 9, the tungsten trioxide A1 was white, and had a reflectivity within the range of 68-86% to visible light within the wavelength range of 400-750 nm.

### Example 2

(1) The certain amounts of phosphotungstic acid, sodium hexadecylbenzene sulfonate and lithium sulfate were weighted, the certain amount of dilute sulfuric acid with a concentration of 0.5M was measured and taken, wherein the specific weighed and measured amounts were shown in Table 1.
(2) Phosphotungstic acid was dissolved in 20 mL of deionized water to prepare a phosphotungstic acid solution, dilute sulfuric acid with a concentration of 0.5M was added into the phosphotungstic acid solution and stirred for 20min, wherein the adding speed of the dilute hydrochloric acid was 7 mL/min. After the addition of dilute sulfuric acid was complete, sodium hexadecylbenzene sulfonate and lithium sulfate were added into the system, and continuously stirred for 25 min to obtain a mixed solution.
(3) The stirred solution was transferred to a hydrothermal reaction kettle with polytetrafluoroethylene as an inner lining and stainless steel as an outer protective sleeve, the hydrothermal reaction kettle was screwed tightly, the hydrothermal reaction was then performed at 180°C for 20 h, and naturally cooled to room temperature.
(4) The reaction product was subjected to centrifugal separation, then washed with deionized water and absolute ethyl alcohol for multiple times until the pH of an eluate was within a range of 6.5-7, the reaction product was subjected to drying in the baking oven at 100°C for 9h to obtain tungsten trioxide A2.

The crystalline phase of tungsten trioxide A2 was hexagonal-phase. In addition, a diffraction peak of 2θ at around 28° was attributed to the (002) crystal plane, a diffraction peak of 2θ at around 22° was attributed to the (100) crystal plane, and a diffraction peak of 2θ at around 14° was attributed to the (001) crystal plane. The relative intensities of the three characteristic diffraction peaks satisfied the following conditions: I₍₀₀₂₎=0.95×I₍₁₀₀₎=1.35×I₍₀₀₁₎.

As illustrated by the SEM analysis, the TEM analysis, and the HRTEM analysis of tungsten trioxide A2, the micro-morphology of the tungsten trioxide A2 was a nanorod bundle formed by stacking a plurality of nanorods with a diameter about 6nm, the nanorod bundle had a length about 800nm and a diameter about 60nm, a length-diameter ratio of the nanorod bundle was 13.33.

The color analysis and the reflection spectrum analysis were performed on the tungsten trioxide A3, the color of tungsten trioxide A2, the reflectivity range for visible light within the wavelength range of 400-750nm, the reflectivity range for blue light within the wavelength range of 400-500nm, and the reflectivity range for red light within the wavelength range of 600-750nm were shown in Table 2.

### Example 3

(1) The certain amounts of sodium phosphotungstate, sodium hexadecylbenzene sulfonate and potassium bisulfate were weighted, the certain amount of the dilute sulfuric acid with the concentration of 3.5M was measured and taken, wherein the specific weighed and measured amounts were shown in Table 1.
(2) Sodium phosphotungstate was dissolved in 65 mL of deionized water to prepare a sodium phosphotungstate solution, dilute sulfuric acid with a concentration of 3.5M was added into the sodium phosphotungstate solution and stirring for 22min, wherein the adding speed of the dilute sulfuric acid was 0.8 mL/min. After the addition of dilute sulfuric acid was complete, sodium hexadecylbenzene sulfonate and potassium bisulfate were added into the system, and continuously stirred for 35 min to obtain a mixed solution.
(3) The stirred solution was transferred to a hydrothermal reaction kettle with polytetrafluoroethylene as an inner lining and stainless steel as an outer protective sleeve, the hydrothermal reaction kettle was screwed tightly, the hydrothermal reaction was then performed at 150°C for 22 h, and naturally cooled to room temperature.
(4) The reaction product was subjected to centrifugal separation, then washed with deionized water and absolute ethyl alcohol for multiple times until the pH of an eluate was within a range of 6.5-7, the reaction product was subjected to drying in the baking oven at 85°C for 25h to obtain tungsten trioxide A3.

The crystalline phase of tungsten trioxide A3 was hexagonal-phase. In addition, a diffraction peak of 2θ at around 28° was attributed to the (002) crystal plane, a diffraction peak of 2θ at around 22° was attributed to the (100) crystal plane, and a diffraction peak of 2θ at around 14° was attributed to the (001) crystal plane. The relative intensities of the three characteristic diffraction peaks satisfied the following conditions: I₍₀₀₂₎=1.02×I₍₁₀₀₎=1.55×I₍₀₀₁₎.

As illustrated by the SEM analysis, the TEM analysis, and the HRTEM analysis of tungsten trioxide A3, the micro-morphology of the tungsten trioxide A3 was a nanorod bundle formed by stacking a plurality of nanorods with a diameter about 5nm, the nanorod bundle had a length about 650nm and a diameter about 55nm, a length-diameter ratio of the nanorod bundle was 11.82.

The color analysis and the reflection spectrum analysis were performed on the tungsten trioxide A3, the color of tungsten trioxide A3, the reflectivity range for visible light within the wavelength range of 400-750nm, the reflectivity range for blue light within the wavelength range of 400-500nm, and the reflectivity range for red light within the wavelength range of 600-750nm were shown in Table 2.

### Comparative Example 1

The preparation method of Comparative Example 1 was the same as the preparation method of Example 3, except that the dilute sulfuric acid was not added.

### Tungsten trioxide DA1 was prepared.

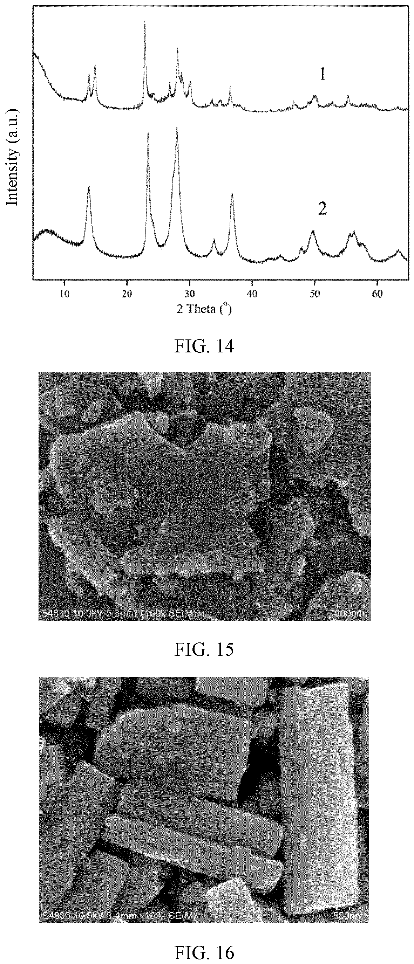

The tungsten trioxide DA1 obtained in Comparative Example 1 was subjected to analysis of crystalline phase, micro-morphology and reflection spectrum, the results were shown in FIG. 14, FIG. 15, FIG. 16 and Table 2. FIG. 14 illustrated the XRD spectrogram of the tungsten trioxide DA1 and DA2 prepared in Comparative Examples 1 and 2, as shown in FIG. 14, the crystalline phase of tungsten trioxide DA1 has diffraction peaks of other impurities in addition to the hexagonal-phase, i.e., the product had a mixed crystalline phase. FIG. 15 showed the SEM analysis results of tungsten trioxide DA1. As illustrated in FIG. 15, the crystalline phase was the irregular morphology mainly in the form of nanoplates. The reflection spectrum analysis of the tungsten trioxide DA1 was carried out, its reflectivity to the light at the wavelength of 470nm was 35%, its reflectivity to the light at the wavelength of 700nm was 22%, its reflectivity to visible light within a wavelength range of 400-750nm was within a range of 21-35%.

The color of tungsten trioxide DA1, the reflectivity range for visible light within the wavelength range of 400-750nm, the reflectivity range for blue light within the wavelength range of 400-500nm, and the reflectivity range for red light within the wavelength range of 600-750nm were shown in Table 2.

### Comparative Example 2

(1) The certain amounts of ammonium metatungstate, citric acid and lithium nitrate were weighed, the certain amount of dilute nitric acid with the concentration of 1.2M was measured and taken, wherein the specific weighed and measured amounts were shown in Table 1.
(2) Ammonium metatungstate was dissolved in 50 mL of deionized water to prepare an ammonium metatungstate solution, dilute nitric acid with a concentration of 1.2M was added into the ammonium metatungstate solution and stirred for 20min, wherein the adding speed of the dilute nitric acid was 4 mL/min. After the addition of dilute sulfuric acid was complete, citric acid and lithium nitrate were added into the system, and continuously stirred for 20 min to obtain a mixed solution.
(3) The stirred solution was transferred to a hydrothermal reaction kettle with polytetrafluoroethylene as an inner lining and stainless steel as an outer protective sleeve, the hydrothermal reaction kettle was screwed tightly, the hydrothermal reaction was then performed at 200°C for 9h, and naturally cooled to room temperature.
(4) The reaction product was subjected to centrifugal separation, then washed with deionized water and absolute ethyl alcohol for multiple times until the pH of an eluate was within a range of 6.5-7, the reaction product was subjected to drying in the baking oven at 90°C for 16h to obtain tungsten trioxide DA2.

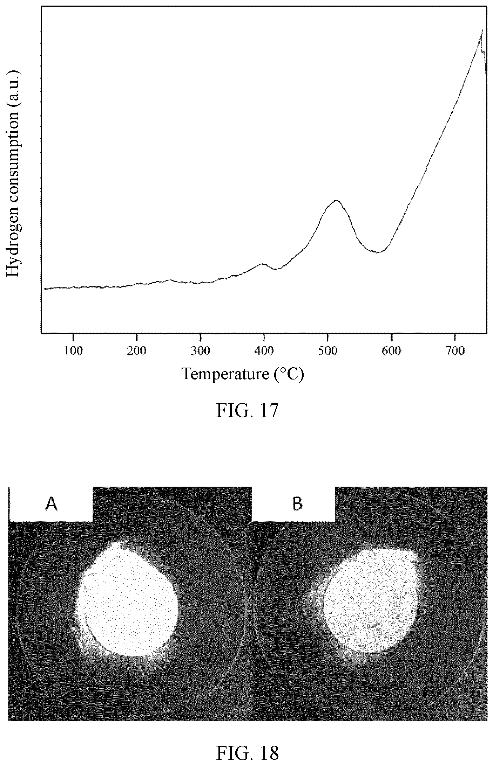

The analysis of crystalline phase, micro-morphology and reflection spectrum was performed on the tungsten trioxide DA2, the analysis results were shown in FIG. 14 and FIG. 17 and Table 2. FIG. 14 illustrated the XRD spectrogram of the tungsten trioxide DA1 and DA2 prepared in Comparative Examples 1 and 2, as shown in FIG. 14, the crystalline phase of tungsten trioxide DA2 was the hexagonal-phase, the JCPDS card number was 35-1001, indicating that it was a different subphase from the hexagonal-phase in Example A1. FIG. 16 showed the SEM analysis results of tungsten trioxide DA1. As shown in FIG. 16, the tungsten trioxide DA2 had a micro-morphology mainly composed of nanorods, its length was within a range of 200-1,000nm, its width was within a range of 100-300 nm, and some nanoparticles were attached thereon.

The tungsten trioxide DA2 was blue, the reflection spectrum of the tungsten trioxide DA2 showed that its reflectivity to the light at the wavelength of 435nm was 32%, its reflectivity to the light at the wavelength of 700nm was 28%, its reflectivity to visible light within a wavelength range of 400-750nm was within a range of 26-36%.

The color of tungsten trioxide DA2, the reflectivity range for visible light within the wavelength range of 400-750nm, the reflectivity range for blue light within the wavelength range of 400-500nm, and the reflectivity range for red light within the wavelength range of 600-750nm were shown in Table 2.

**Table 1**

| | Tungsten trioxide | Tungsten salt | Inorganic acid | Organic additive | Structure-directing agent | Molar ratio ¹ | Proportion ² (%) | Mass ratio 3 |
|---|---|---|---|---|---|---|---|---|
| Example 1 | A1 | 4.5g Sodium tungstate | 2.5 M diluted hydrochloric acid 15 mL | 1.125g Oxalic acid | 6.75g Sodium sulfate | 0.27:1 | 25% | 1:1.5 |
| Example 2 | A2 | 3.7g phosphotungstic acid | 0.5 M Diluted sulfuric acid 49 mL | 0.74g Sodium hexadecylbenzene sulfonate | 4.62g Lithium sulfate | 0.58:1 | 8% | 0.8:1 |
| Example 3 | A3 | 7.3g Sodium phosphotungstate | 3.5 M Diluted sulfuric acid 3.8 mL | 2.55g Sodium hexadecylbenzene sulfonate | 5.62g Potassium bisulfate | 2.86:1 | 77% | 1.3:1 |
| Comparative Example 1 | DA1 | 7.3g Sodium phosphotungstate | - | 2.55g Sodium hexadecylbenzene sulfonate | 5.62g Potassium bisulfate | 4.3:1 | 100% | 1.3:1 |
| Comparative Example 2 | DA2 | 5.94g Ammonium metatungstate | 1.2 M diluted nitric acid 12 mL | 1.74g Citric acid | 3.96g Lithium nitrate | 1:1 | 38% | 1.5:1 |

| | | | | | | | | |
|---|---|---|---|---|---|---|---|---|
| Note: 1 - The molar ratio of tungsten salt: (the sum of organic additive and inorganic acid); 2 - The proportion (%) of the organic additive to the total mole number of the organic additive and the inorganic acid; 3 - The mass ratio of tungsten salt: structure-directing agent | | | | | | | | |

**Table 2**

| | Tungsten trioxide | Color | Micro-morpholo gy | Crystalline phase | Reflectivity | | |
|---|---|---|---|---|---|---|---|
| | | | | | 400-750 nm | 400-500nm | 600-750nm |
| Example 1 | A1 | White | Nanorod bundle | Hexagonal-phase | 68-86% | 68-86% | 79-81% |
| Example 2 | A2 | White | Nanorod bundle | Hexagonal-phase | 58-81% | 58-81% | 75-79% |
| Example 3 | A3 | White | Nanorod bundle | Hexagonal-phase | 51-77% | 51-77% | 72-75% |
| Comparative Example 1 | DA 1 | Blue | Irregular morphology mainly in the form of nanoplates | Mixed crystalline phase | 21-35% | 31-35% | 21-26% |
| Comparative Example 2 | DA2 | Blue | Irregular nanorod | Hexagonal-phase (subphase) (JCPDS: 35-1001) | 26-36% | 32-36% | 26-30% |

As can be seen from Table 2, the micro-morphology of the tungsten trioxide prepared in Examples 1-3 was a nanorod bundle, the crystalline phase was a hexagonal-phase, and its reflectivity to visible light within the wavelength range of 400-750nm was larger than 50%, a white color can be observed. The micro-morphologies of the tungsten trioxide prepared in Comparative Examples 1 and 2 were not a nanorod bundle, and the reflectivity to visible light within the wavelength range of 400-750nm was less than 40%, the reflectivity to blue light within the wavelength range of 400-500nm was within a range of 30-37%, and the reflectivity to red light within the wavelength range of 600-750nm was within a range of 20-30%, and the blue color can be observed.

### Example 4

(1) 3 g of tungsten trioxide A1 and 0.03 g of chloroplatinic acid were accurately weighted, wherein the addition amount of the chloroplatinic acid was 1 wt.% of the addition amount of tungsten trioxide, the tungsten trioxide A1 and the chloroplatinic acid were added into 50 mL of deionized water and stirred for 10 min to obtain a solid-liquid mixture;
(2) 7.5 g of ascorbic acid was weighed, the addition mass of the ascorbic acid was 2.5 times of tungsten trioxide. The ascorbic acid was added into the solid-liquid mixture and continuously stirred for 20 min, the mixture was then transferred to a hydrothermal reaction kettle with polytetrafluoroethylene as an inner lining and stainless steel as an outer protective sleeve, the hydrothermal reaction kettle was screwed tightly, the hydrothermal reaction was then performed at 150°C for 5 h, and naturally cooled to room temperature;
(3) The reaction product was subjected to centrifugal separation, then washed with deionized water and absolute ethyl alcohol for multiple times until the pH of an eluate was within a range of 6.5-7, the reaction product was subjected to drying in the baking oven at 85°C for 12h to obtain the hydrogen leakage detecting material B1.

X-ray fluorescence analysis was performed on the hydrogen leakage detecting material B1, the content of the platinum nanoparticles on the hydrogen leakage detecting material was detected to be 0.35 wt.%.

XRD analysis, TPR analysis, SAED analysis, HRTEM analysis were performed on the hydrogen leakage detecting material prepared in Example 4, the results were shown in FIGS. 1-2 and FIGS. 6-7.

FIG. 1 illustrated the XRD analysis results of the hydrogen leakage detecting material B 1, the reference sign 1 showed the XRD analysis results of the hydrogen leakage detecting material B1, and the reference sign 2 showed the XRD analysis results of tungsten trioxide A1. By comparing with the standard card JCPDS No.33-1387, it was found that all peaks could be attributed to their color-changing substrate tungsten trioxide A1, and the diffraction peaks attributed to the platinum species were not detected. It indicated that the platinum species had small nanometer size, which exceeded the lower limit (2-5 nm) of XRD analysis and detection.

FIG. 2 showed the TPR analysis results of the hydrogen leakage detecting material B1, as can be seen, tungsten trioxide A1 lacked the H₂ desorption capability below the temperature 350°C, the temperature of the obvious hydrogen consumption peak was 490°C, while the temperature of the obvious hydrogen consumption peak of the tungsten trioxide A1 was 72°C, the starting temperature of the obvious hydrogen consumption peak was 46°C, which demonstrated that the hydrogen leakage detecting material B1 had the desorption capability to hydrogen gas at 46°C, namely the starting reduction temperature to hydrogen gas was 46°C, which was reduced to below 50°C. It indicated that the hydrogen leakage detecting material B1 had significant low-temperature reduction performance, thereby facilitating the hydrogen gas desorption and hydrogen-sensitive color change reaction at the low temperature or even at room temperature.

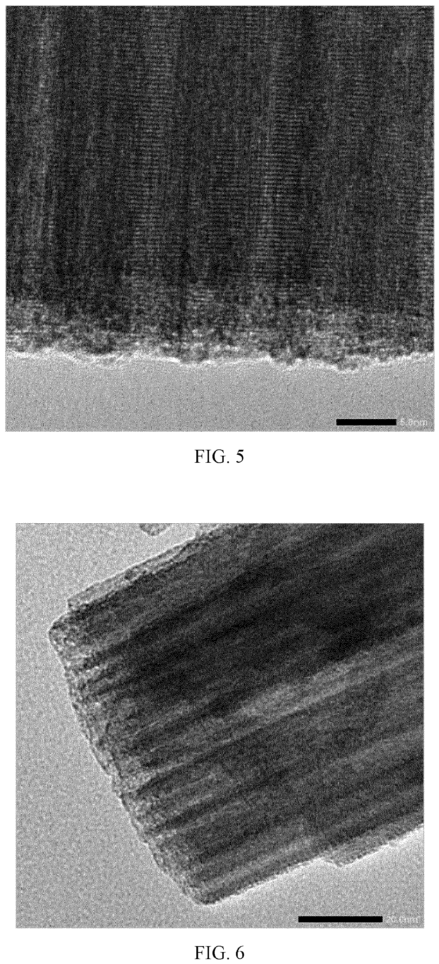

FIG. 6 showed an HRTEM photograph of the hydrogen leakage detecting material B1, as can be seen, the microstructure of the hydrogen leakage detecting material B1 was the same as that of tungsten trioxide A1, and the original nanotube bundle microstructure was maintained, which indicated that the original microstructure of tungsten trioxide was not changed after introducing the noble metal, and the noble metal nanoparticles were highly dispersed and the obvious particle agglomeration did not occur.

FIG. 7 illustrated an element distribution photograph of the hydrogen leakage detecting material B1, as can be seen, the platinum metal was highly uniformly dispersed on the tungsten trioxide A1, and the platinum nanoparticle size was less than 2nm.

Hydrogen gas having a H₂ concentration of 10 vol.% was introduced at a flow rate of 100mL/min at room temperature for a time of introducing hydrogen gas being 60s, the reflection spectrum analysis and color analysis were performed on the hydrogen leakage detecting material B1 before and after introducing hydrogen gas, and the results were shown in FIG. 8 and FIG. 9. The curves 2 and 3 in FIG. 8 represented the reflection spectra of the hydrogen leak material B1 before and after introducing hydrogen gas, respectively, FIG. 9B and FIG. 9C illustrated the photographs comparing the hydrogen leak detection material B1 before and after introducing hydrogen gas, respectively. As illustrated by FIG. 8, before introducing hydrogen gas into the hydrogen leakage detecting material B1, the reflectivity to visible light in the range of 400-750nm was high and within the range of 45-56%, the reflectivity to visible light within the range of 400-500nm was greater than or equal to 45%, and the reflectivity to visible light within the range of 600-750nm was greater than or equal to 55%, which was consistent with the gray color of the hydrogen leakage detecting material B1 in FIG. 9B. After introducing the hydrogen gas with a concentration of 10vol.% for 60s, the reflectivity of the hydrogen leakage detecting material was greatly reduced, the reflectivity to the visible light within the wavelength range of 400-750nm was within a range of 4.9-27%, wherein the reflectivity of blue light within the wavelength range of 400-500nm was relatively high, the reflectivity was within a range of 22-27%, but the reflectivity to red light within the wavelength range of 600-750nm was lower, the reflectivity was within a range of 4.9-9%, that is, the reflected light of the hydrogen leakage detecting material B1 after introducing the hydrogen gas was mainly the blue light, such as the deep blue light. Furthermore, as shown by FIG. 8, the lowest value of the reflectivity change difference of the hydrogen leakage detection material B1 to visible light within the wavelength range of 400-750nm before and after introducing H₂ was greater than 22%, the lowest value of the reflectivity change difference of the hydrogen leakage detection material B1 to blue light within the wavelength range of 400-500nm before and after introducing H₂ was greater than 22%, the lowest value of the reflectivity change difference of the hydrogen leakage detection material B 1 to red light within the wavelength range of 600-750nm before and after introducing H₂ was greater than 40%, which was corresponding to the significant color change before and after introducing the hydrogen gas. As shown in FIG. 9, it can be observed that the color of the hydrogen leakage detecting material B1 before introducing hydrogen gas was gray white, and the color was dark blue after introducing hydrogen gas, the color contrast was significant. During the process of introducing hydrogen gas having a H₂ concentration of 10 vol.% into the hydrogen leakage detecting material B1 at a flow rate of 100mL/min at room temperature, the reflection spectrum of the hydrogen leakage detecting material B1 was detected in real time, and FIG. 10 was drawn to illustrate the reflectivity change of the material B1 to the light at wavelengths of 475nm and 700nm along with time. As can be seen from FIG. 10, after hydrogen gas was introduced for 5 s, the reflectivity of the hydrogen leakage detecting material B1 to the light at the wavelength of 475nm was reduced from 53.8% to 53.2%, and the reflectivity change difference was 0.6%; the reflectivity to the light at the wavelength of 700nm was decreased from 56% to 47.7%, the reflectivity change difference was 8.3%, as can be seen, after introducing hydrogen gas, the reflectivity decrease of the hydrogen leakage detecting material B1 for blue light was more slowly than the reflectivity decrease for red light, the material reflected more blue light, which indicates that the color of the material changed to blue color. In addition, after hydrogen gas was introduced into the light source detecting material, it consumed about 6.6s that the reflectivity change difference to light at a wavelength of 700nm reached 10% (i.e., the reflectivity was reduced from 56% to 46%), that is, a remarkable color change can be observed after 6.6s.

The dispersity analysis was performed on the hydrogen leakage detecting material B1, the result showed that the dispersity of metal platinum on tungsten trioxide was 76%.

The hydrogen leakage detection was performed on the hydrogen leakage detecting material B1, the specific results were shown in FIGS. 11-13 and Tables 3-5 as follows:
1. FIG. 11 showed the detection results of the color-changing response time of the hydrogen leakage detecting material B1 for hydrogen gas having different H₂ concentrations at a flow rate of 200 mL/min at room temperature. As shown in FIG. 11, the hydrogen leakage detecting material had a color-changing response time of less than or equal to 2s for pure hydrogen gas; when the hydrogen gas concentration was 4 vol.%, the color-changing response time of the hydrogen leakage detecting material was less than 8s; when the hydrogen gas concentration was 1 vol.%, the color-changing response time of the hydrogen leakage detecting material was less than 15s; when the hydrogen gas concentration was 0.1 vol.%, the color-changing response time of the hydrogen leakage detecting material was about 35s.

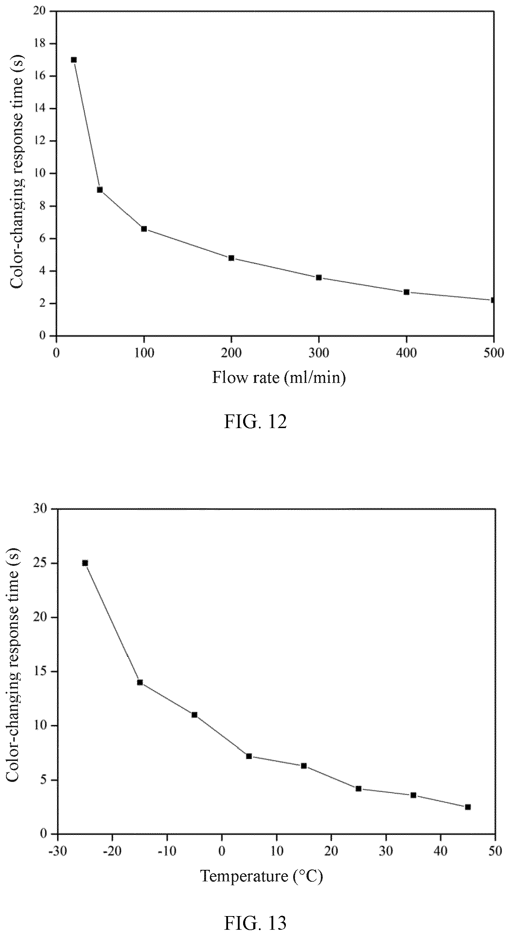

2. FIG. 12 illustrated the color-changing response time of the hydrogen leakage detecting material B1 for hydrogen gas having a H₂ concentration of 10vol.% at different flow rates at room temperature. As shown in FIG. 12, the hydrogen leakage detecting material B1 had the color-changing response time of less than 3s for hydrogen gas at a flow rate of 500mL/min, and the hydrogen leakage detecting material B1 had the color-changing response time of less than 5s for hydrogen gas at a flow rate of 200mL/min.
3. FIG. 13 illustrated the color-changing response time of the hydrogen leakage detecting material B1 for hydrogen gas at different gas temperatures with a flow rate of 200mL/min and a H₂ concentration of 10 vol.%. As shown in FIG. 13, the color-changing response time of the hydrogen leakage detecting material B1 was about 25s for hydrogen gas at -25°C, the color-changing response time of the hydrogen leakage detecting material B 1 was less than 8s for hydrogen gas at 5°C, and the color-changing response time of the hydrogen leakage detecting material B1 was less than 5s for hydrogen gas at 25°C.

As can be seen from the results of the hydrogen leakage detection test, the hydrogen leakage detecting material B1 had a high sensitivity to hydrogen gas under different conditions, the color change speed at room temperature was high, reached the quick response level of several seconds, and the color-changing response time did not exceed 1 min even at the low temperature.

### Example 5

(1) 4.5g of tungsten trioxide A2 and 0.07g of platinum(II) nitrate were accurately weighted, wherein the addition amount of the platinum(II) nitrate was 1.5 wt.% of the addition amount of tungsten trioxide, the tungsten trioxide A2 and the platinum(II) nitrate were added into 50 mL of deionized water and stirred for 10 min to obtain a solid-liquid mixture;
(2) 12 mL of pure ethylene glycol was weighted, the addition mass of the ethylene glycol was 3 times of the addition mass of tungsten oxide. The ethylene glycol was added into the solid-liquid mixture and continuously stirred for 20 min, the mixture was then transferred to a hydrothermal reaction kettle with polytetrafluoroethylene as an inner lining and stainless steel as an outer protective sleeve, the hydrothermal reaction kettle was screwed tightly, the hydrothermal reaction was then performed at 155°C for 4h, and naturally cooled to room temperature to obtain a reaction product;
(3) The reaction product was subjected to centrifugal separation, then washed with deionized water and absolute ethyl alcohol for multiple times until the pH of an eluate was within a range of 6.5-7.0, the reaction product was subjected to drying in the baking oven at 70°C for 24h to obtain the hydrogen leakage detecting material B2.

X-ray fluorescence analysis was performed on the hydrogen leakage detecting material B2, the content of the platinum nanoparticles on the hydrogen leakage detecting material was detected to be 0.9 wt.%.

The HRTEM image of the hydrogen leakage detecting material B2 showed that the microstructure was the same as that of tungsten trioxide A2, and maintained the original nanotube bundle microstructure.

As can be seen from XRD analysis of the hydrogen leakage detecting material B2, all peaks of the hydrogen leakage detecting material B2 could be attributed to its color-changing substrate tungsten trioxide A2, and the diffraction peaks attributed to the platinum species were not detected. It indicated that the platinum species had small nanometer size, which exceeded the lower limit (2-5 nm) of XRD analysis and detection.

According to an analysis on the element distribution photograph of the hydrogen leakage detecting material B2, the platinum metal was highly uniformly dispersed on the tungsten trioxide A2, and the platinum nanoparticle size was less than 2nm.

Before hydrogen gas was introduced to the hydrogen leakage detecting material B2, the reflection spectrum detection analysis was performed on the hydrogen leakage detecting material B2, the reflection spectrum of the hydrogen leakage detecting material B2 was observed to be similar to that of line 2 in FIG. 8 in Example 4. The reflectivity of the hydrogen leakage detecting material B2 to visible light within the wavelength range of 400-750nm was 45-52%, the reflectivity to blue light within the wavelength range of 400-500nm was larger than or equal to 45%, and the reflectivity to red light within the range of 600-750nm was larger than or equal to 50%.

Hydrogen gas having a H₂ concentration of 10vol.% was introduced at the flow rate of 100mL/min at room temperature for 60s, the reflection spectrum detection analysis was performed on the hydrogen leakage detecting material B2 at the moment, the reflection spectrum was observed to be similar to that of the line 3 in FIG. 8 in Example 4, the reflectivity range of the hydrogen leakage detecting material B2 to visible light within the wavelength range of 400-750nm was 5-26%, the reflectivity range of the hydrogen leakage detecting material B2 to blue light within the wavelength range of 400-500nm was 22-26%, and the reflectivity range of the hydrogen leakage detecting material B2 to red light within the range of 600-750nm was 5-9%. The lowest value of the reflectivity change difference of the hydrogen leakage detection material B2 to visible light within the wavelength range of 400-750nm before and after introducing H₂ was greater than 22%, the lowest value of the reflectivity change difference of the hydrogen leakage detection material B2 to blue light within the wavelength range of 400-500nm before and after introducing H₂ was greater than 22%, the lowest value of the reflectivity change difference of the hydrogen leakage detection material B2 to red light within the wavelength range of 600-700nm before and after introducing H₂ was greater than 42%.

The colors of the hydrogen leakage detecting material B2 before and after introducing hydrogen gas were observed to be similar with the colors of the hydrogen leakage detecting material B1 before and after introducing hydrogen gas.

The results of TPR analysis, the results of dispersity analysis, and the results of partial hydrogen leakage detection on the hydrogen leakage detecting material B2 were shown in Table 6.

The hydrogen leakage detection was performed on the hydrogen leakage detecting material B2, specifically:
1. The detection results of color-changing response time of the hydrogen leakage detecting material B2 to hydrogen gas having different H₂ concentrations at a flow rate of 200mL/min at room temperature were shown in Table 3.
2. The detection results of color-changing response time of the hydrogen leakage detecting material B2 to hydrogen gas having a H₂ concentration of 10vol.% at different flow rates were shown in Table 4.
3. The detection results of color-changing response time of the hydrogen leakage detecting material B2 to hydrogen gas having a H₂ concentration of 10vol.% at a flow rate of 200mL/min at various gas temperatures were shown in Table 5.

### Example 6

(1) 6.2g of tungsten trioxide A1 and 0.04g of chloroplatinic acid were accurately weighted, wherein the addition amount of the chloroplatinic acid was 0.65 wt.% of the addition amount of tungsten trioxide, the tungsten trioxide A1 and the chloroplatinic acid were added into 55mL of deionized water and stirring for 10 min to obtain a solid-liquid mixture;
(2) 22 mL of pure ethylene glycol was weighted, the addition mass of ethylene glycol was 3.9 times the addition mass of tungsten oxide. The ethylene glycol was added into the solid-liquid mixture and continuously stirred for 20 min, the mixture was then transferred to a hydrothermal reaction kettle with polytetrafluoroethylene as an inner lining and stainless steel as an outer protective sleeve, the hydrothermal reaction kettle was screwed tightly, the hydrothermal reaction was then performed at 135°C for 3.2h, and naturally cooled to room temperature to obtain a reaction product;
(3) The reaction product was subjected to centrifugal separation, then washed with deionized water and absolute ethyl alcohol for multiple times until the pH of an eluate was within a range of 6.5-7.0, the reaction product was subjected to drying in the baking oven at 70°C for 24h to obtain the hydrogen leakage detecting material B3.

X-ray fluorescence analysis was performed on the hydrogen leakage detecting material B3, the content of the platinum nanoparticles on the hydrogen leakage detecting material was detected to be 0.24 wt.%.

The HRTEM image of the hydrogen leakage detecting material B3 showed that the microstructure was the same as that of tungsten trioxide A1, and maintained the original nanotube bundle microstructure.

As can be seen from XRD analysis of the hydrogen leakage detecting material B3, all peaks of the hydrogen leakage detecting material B2 could be attributed to its color-changing substrate tungsten trioxide A1, and the diffraction peaks attributed to the platinum species were not detected. It indicated that the platinum species had small nanometer size, which exceeded the lower limit (2-5 nm) of XRD analysis and detection.

According to an analysis on the element distribution photograph of the hydrogen leakage detecting material B3, the platinum metal was highly uniformly dispersed on the tungsten trioxide A1, and the platinum nanoparticle size was less than 2nm.

Before hydrogen gas was introduced to the hydrogen leakage detecting material B3, the reflection spectrum detection analysis was performed on the hydrogen leakage detecting material B3, the reflection spectrum of the hydrogen leakage detecting material B3 was observed to be similar to that of line 2 in FIG. 8 in Example 4. The reflectivity of the hydrogen leakage detecting material B3 to visible light within the wavelength range of 400-750nm was 46-56%, the reflectivity to blue light within the wavelength range of 400-500nm was larger than or equal to 46%, and the reflectivity to red light within the range of 600-750nm was larger than or equal to 55%.

Hydrogen gas having a H₂ concentration of 10vol.% was introduced at the flow rate of 100mL/min at room temperature for 60s, the reflection spectrum detection analysis was performed on the hydrogen leakage detecting material B3 at the moment, the reflection spectrum was observed to be similar to that of the line 3 in FIG. 8 in Example 4, the reflectivity range of the hydrogen leakage detecting material B3 to visible light within the wavelength range of 400-750nm was 5-26%, the reflectivity range of the hydrogen leakage detecting material B3 to blue light within the wavelength range of 400-500nm was 21-26%, and the reflectivity range of the hydrogen leakage detecting material B3 to red light within the range of 600-750nm was 5-10%. The lowest value of the reflectivity change difference of the hydrogen leakage detection material B3 to visible light within the wavelength range of 400-750nm before and after introducing H₂ was greater than 23%, the lowest value of the reflectivity change difference of the hydrogen leakage detection material B3 to blue light within the wavelength range of 400-500nm before and after introducing H₂ was greater than 23%, the lowest value of the reflectivity change difference of the hydrogen leakage detection material B3 to red light within the wavelength range of 600-750nm before and after introducing H₂ was greater than 36%.

The colors of the hydrogen leakage detecting material B3 before and after introducing hydrogen gas were observed to be similar with the colors of the hydrogen leakage detecting material B1 before and after introducing hydrogen gas.

The results of TPR analysis, the results of dispersity analysis, and the results of partial hydrogen leakage detection on the hydrogen leakage detecting material B3 were shown in Table 6.

The hydrogen leakage detection was performed on the hydrogen leakage detecting material B3, specifically:
1. The detection results of color-changing response time of the hydrogen leakage detecting material B3 to hydrogen gas having different H₂ concentrations at a flow rate of 200mL/min at room temperature were shown in Table 3
2. The detection results of color-changing response time of the hydrogen leakage detecting material B3 to hydrogen gas having a H₂ concentration of 10vol.% at different flow rates were shown in Table 4.
3. The detection results of color-changing response time of the hydrogen leakage detecting material B3 to hydrogen gas having a H₂ concentration of 10vol.% at a flow rate of 200mL/min at various gas temperatures were shown in Table 5.

### Example 7

(1) 5.7g of tungsten trioxide A1 and 0.068g of platinum(II) nitrate were accurately weighted, wherein the addition amount of the platinum(II) nitrate was 1.2 wt.% of the addition amount of tungsten trioxide, the tungsten trioxide A1 and the platinum(II) nitrate were added into 50mL of deionized water and stirred for 10 min to obtain a solid-liquid mixture;
(2) 15.5g of ascorbic acid was weighted, the addition mass of ascorbic acid was 2.7 times the addition mass of tungsten oxide. The ascorbic acid was added into the solid-liquid mixture and continuously stirred for 20 min, the mixture was then transferred to a hydrothermal reaction kettle with polytetrafluoroethylene as an inner lining and stainless steel as an outer protective sleeve, the hydrothermal reaction kettle was screwed tightly, the hydrothermal reaction was then performed at 125°C for 5.5h, and naturally cooled to room temperature to obtain a reaction product;
(3) The reaction product was subjected to centrifugal separation, then washed with deionized water and absolute ethyl alcohol for multiple times until the pH of an eluate was within a range of 6.5-7.0, the reaction product was subjected to drying in the baking oven at 70°C for 24h to obtain the hydrogen leakage detecting material B4.

X-ray fluorescence analysis was performed on the hydrogen leakage detecting material B4, the content of the platinum nanoparticles on the hydrogen leakage detecting material was detected to be 0.62 wt.%.

The HRTEM image of the hydrogen leakage detecting material B4 showed that the microstructure was the same as that of tungsten trioxide A1, and maintained the original nanotube bundle microstructure.

As can be seen from XRD analysis of the hydrogen leakage detecting material B4, all peaks of the hydrogen leakage detecting material B4 could be attributed to its color-changing substrate tungsten trioxide A1, and the diffraction peaks attributed to the platinum species were not detected. It indicated that the platinum species had small nanometer size, which exceeded the lower limit (2-5 nm) of XRD analysis and detection.

According to an analysis on the element distribution photograph of the hydrogen leakage detecting material B4, the platinum metal was highly uniformly dispersed on the tungsten trioxide A1, and the platinum nanoparticle size was less than 2nm.

Before hydrogen was introduced to the hydrogen leakage detecting material B4, the reflection spectrum detection analysis was performed on the hydrogen leakage detecting material B4, the reflection spectrum of the hydrogen leakage detecting material B4 was observed to be similar to that of line 2 in FIG. 8 in Example 4. The reflectivity of the hydrogen leakage detecting material B4 to visible light within the wavelength range of 400-750nm was 45-55%, the reflectivity to blue light within the wavelength range of 400-500nm was larger than or equal to 45%, and the reflectivity to red light within the range of 600-750nm was larger than or equal to 54%.

Hydrogen gas having a H₂ concentration of 10vol.% was introduced at the flow rate of 100mL/min at room temperature for 60s, the reflection spectrum detection analysis was performed on the hydrogen leakage detecting material B4 at the moment, the reflection spectrum was observed to be similar to that of the line 3 in FIG. 8 in Example 4, the reflectivity range of the hydrogen leakage detecting material B4 to visible light within the wavelength range of 400-750nm was 4.5-26%, the reflectivity range of the hydrogen leakage detecting material B4 to blue light within the wavelength range of 400-500nm was 20-26%, and the reflectivity range of the hydrogen leakage detecting material B4 to red light within the range of 600-750nm was 4.5-9%. The lowest value of the reflectivity change difference of the hydrogen leakage detection material B4 to visible light within the wavelength range of 400-750nm before and after introducing H₂ was greater than 22%, the lowest value of the reflectivity change difference of the hydrogen leakage detection material B4 to blue light within the wavelength range of 400-500nm before and after introducing H₂ was greater than 22%, the lowest value of the reflectivity change difference of the hydrogen leakage detection material B4 to red light within the wavelength range of 600-750nm before and after introducing H₂ was greater than 36%.

The colors of the hydrogen leakage detecting material B4 before and after introducing hydrogen gas were observed to be similar with the colors of the hydrogen leakage detecting material B1 before and after introducing hydrogen gas.

The results of TPR analysis, the results of dispersity analysis, and the results of partial hydrogen leakage detection on the hydrogen leakage detecting material B4 were shown in Table 6.

The hydrogen leakage detection was performed on the hydrogen leakage detecting material B4, specifically:
1. The detection results of color-changing response time of the hydrogen leakage detecting material B4 to hydrogen gas having different H₂ concentrations at a flow rate of 200mL/min at room temperature were shown in Table 3.
2. The detection results of color-changing response time of the hydrogen leakage detecting material B4 to hydrogen gas having a H₂ concentration of 10vol.% at different flow rates were shown in Table 4.
3. The detection results of color-changing response time of the hydrogen leakage detecting material B4 to hydrogen gas having a H₂ concentration of 10vol.% at a flow rate of 200mL/min at various gas temperatures were shown in Table 5.

### Example 8

(1) 3g of tungsten trioxide A1 and 0.045g of chloroplatinic acid were accurately weighted, wherein the addition amount of the chloroplatinic acid was 1.5 wt.% of the addition amount of the tungsten trioxide, the tungsten trioxide A1 and the chloroplatinic acid were added into 50 mL of deionized water and stirred for 10 min to obtain a solid-liquid mixture;
(2) 1.5 g of ascorbic acid and 6 ml of pure ethylene glycol (the mass ratio of the ethylene glycol to the ascorbic acid was 1:0.23) were weighted, the ascorbic acid and the pure ethylene glycol were added into the solid-liquid mixture and continuously stirred for 20 min, the mixture was then transferred to a hydrothermal reaction kettle with polytetrafluoroethylene as an inner lining and stainless steel as an outer protective sleeve, the hydrothermal reaction kettle was screwed tightly, the hydrothermal reaction was then performed at 140°C for 5h, and naturally cooled to room temperature to obtain a reaction product;
(3) The reaction product was subjected to centrifugal separation, then washed with deionized water and absolute ethyl alcohol for multiple times until the pH of an eluate was within a range of 6.5-7, the reaction product was subjected to drying in the baking oven at 85°C for 12h to obtain the hydrogen leakage detecting material B5.

X-ray fluorescence analysis was performed on the hydrogen leakage detecting material B5, the content of the platinum nanoparticles on the hydrogen leakage detecting material was detected to be 0.97 wt.%.

The HRTEM image of the hydrogen leakage detecting material B5 showed that the microstructure was the same as that of tungsten trioxide A1, and maintained the original nanotube bundle microstructure.

As can be seen from XRD analysis of the hydrogen leakage detecting material B5, all peaks of the hydrogen leakage detecting material B5 could be attributed to its color-changing substrate tungsten trioxide A1, and the diffraction peaks attributed to the platinum species were not detected. It indicated that the platinum species had small nanometer size, which exceeded the lower limit (2-5 nm) of XRD analysis and detection.

According to an analysis on the element distribution photograph of the hydrogen leakage detecting material B5, the platinum metal was highly uniformly dispersed on the tungsten trioxide A1, and the platinum nanoparticle size was less than 2nm.

Before hydrogen gas was introduced to the hydrogen leakage detecting material B5, the reflection spectrum detection analysis was performed on the hydrogen leakage detecting material B5, the reflection spectrum of the hydrogen leakage detecting material B5 was observed to be similar to that of line 2 in FIG. 8 in Example 4. The reflectivity of the hydrogen leakage detecting material B5 to visible light within the wavelength range of 400-750nm was 46-53%, the reflectivity to blue light within the wavelength range of 400-500nm was larger than or equal to 46%, and the reflectivity to red light within the range of 600-750nm was larger than or equal to 52%.

Hydrogen gas having a H₂ concentration of 10vol.% was introduced at the flow rate of 100mL/min at room temperature for 60s, the reflection spectrum detection analysis was performed on the hydrogen leakage detecting material B5 at the moment, the reflection spectrum was observed to be similar to that of the line 3 in FIG. 8 in Example 4, the reflectivity range of the hydrogen leakage detecting material B5 to visible light within the wavelength range of 400-750nm was 4.5-26%, the reflectivity range of the hydrogen leakage detecting material B5 to blue light within the wavelength range of 400-500nm was 20-26%, and the reflectivity range of the hydrogen leakage detecting material B5 to red light within the range of 600-700nm was 4.5-9%. The lowest value of the reflectivity change difference of the hydrogen leakage detection material B5 to visible light within the wavelength range of 400-750nm before and after introducing H₂ was greater than 22%, the lowest value of the reflectivity change difference of the hydrogen leakage detection material B5 to blue light within the wavelength range of 400-500nm before and after introducing H₂ was greater than 22%, the lowest value of the reflectivity change difference of the hydrogen leakage detection material B5 to red light within the wavelength range of 600-750nm before and after introducing H₂ was greater than 38%.

The colors of the hydrogen leakage detecting material B5 before and after introducing hydrogen gas were observed to be similar with the colors of the hydrogen leakage detecting material B1 before and after introducing hydrogen.

The results of TPR analysis, the results of dispersity analysis, and the results of partial hydrogen leakage detection on the hydrogen leakage detecting material B5 were shown in Table 6.

The hydrogen leakage detection was performed on the hydrogen leakage detecting material B5, specifically:
1. The detection results of color-changing response time of the hydrogen leakage detecting material B5 to hydrogen gas having different H₂ concentrations at a flow rate of 200mL/min at room temperature were shown in Table 3.
2. The detection results of color-changing response time of the hydrogen leakage detecting material B5 to hydrogen gas having a H₂ concentration of 10vol.% at different flow rates were shown in Table 4.
3. The detection results of color-changing response time of the hydrogen leakage detecting material B5 to hydrogen gas having a H₂ concentration of 10vol.% at a flow rate of 200mL/min at various gas temperatures were shown in Table 5.

### Example 9

The preparation method of Example 9 was the same as that in Example 7, except that the addition amount of platinum(II) nitrate was 2.5wt.% of the addition amount of tungsten trioxide. The hydrogen leakage detecting material B6 was prepared.

X-ray fluorescence analysis was performed on the hydrogen leakage detecting material B6, the content of the platinum nanoparticles on the hydrogen leakage detecting material was detected to be 1.2 wt.%.

The HRTEM image of the hydrogen leakage detecting material B6 showed that the microstructure was the same as that of tungsten trioxide A1, and maintained the original nanotube bundle microstructure.

As can be seen from XRD analysis of the hydrogen leakage detecting material B6, all peaks of the hydrogen leakage detecting material B4 could be attributed to its color-changing substrate tungsten trioxide A1, and the diffraction peaks attributed to the platinum species were not detected. It indicated that the platinum species had small nanometer size, which exceeded the lower limit (2-5 nm) of XRD analysis and detection.

According to an analysis on the element distribution photograph of the hydrogen leakage detecting material B6, the platinum metal was highly uniformly dispersed on the tungsten trioxide A1, and the platinum nanoparticle size was less than 2nm.

Before hydrogen was introduced to the hydrogen leakage detecting material B6, the reflection spectrum detection analysis was performed on the hydrogen leakage detecting material B4, the reflection spectrum of the hydrogen leakage detecting material B6 was observed to be similar to that of line 2 in FIG. 8 in Example 4. The reflectivity of the hydrogen leakage detecting material B6 to visible light within the wavelength range of 400-750nm was 45-52%, the reflectivity to blue light within the wavelength range of 400-500nm was larger than or equal to 45%, and the reflectivity to red light within the range of 600-750nm was larger than or equal to 49%.

Hydrogen gas having a H₂ concentration of 10vol.% was introduced at the flow rate of 100mL/min at room temperature for 60s, the reflection spectrum detection analysis was performed on the hydrogen leakage detecting material B6 at the moment, the reflection spectrum was observed to be similar to that of the line 3 in FIG. 8 in Example 4, the reflectivity range of the hydrogen leakage detecting material B6 to visible light within the wavelength range of 400-750nm was 4-25%, the reflectivity range of the hydrogen leakage detecting material B6 to blue light within the wavelength range of 400-500nm was 19-25%, and the reflectivity range of the hydrogen leakage detecting material B6 to red light within the range of 600-750nm was 4-8%. The lowest value of the reflectivity change difference of the hydrogen leakage detection material B6 to visible light within the wavelength range of 400-750nm before and after introducing H₂ was greater than 22%, the lowest value of the reflectivity change difference of the hydrogen leakage detection material B6 to blue light within the wavelength range of 400-500nm before and after introducing H₂ was greater than 22%, the lowest value of the reflectivity change difference of the hydrogen leakage detection material B6 to red light within the wavelength range of 600-750nm before and after introducing H₂ was greater than 35%.

The colors of the hydrogen leakage detecting material B6 before and after introducing hydrogen gas were observed to be similar with the colors of the hydrogen leakage detecting material B1 before and after introducing hydrogen gas.

The results of TPR analysis, the results of dispersity analysis, and the results of partial hydrogen leakage detection on the hydrogen leakage detecting material B6 were shown in Table 6.

The hydrogen leakage detection was performed on the hydrogen leakage detecting material B6, specifically:
1. The detection results of color-changing response time of the hydrogen leakage detecting material B6 to hydrogen gas having different H₂ concentrations at a flow rate of 200mL/min at room temperature were shown in Table 3.
2. The detection results of color-changing response timeof the hydrogen leakage detecting material B6 to hydrogen gas having a H₂ concentration of 10vol.% at different flow rates were shown in Table 4.
3. The detection results of color-changing response time of the hydrogen leakage detecting material B6 to hydrogen gas having a H₂ concentration of 10vol.% at a flow rate of 200mL/min at various gas temperatures were shown in Table 5.

### Comparative Example 3

The preparation method of Comparative Example 3 was the same as that of Example 4, except that tungsten trioxide DA2 was used as color-changing substrate, thus the hydrogen leakage detecting material DB1 was prepared.

Before hydrogen gas was introduced to the hydrogen leakage detecting material DB1, the reflection spectrum detection analysis was performed on the hydrogen leakage detecting material DB1. It was observed that the reflectivity of the hydrogen leakage detecting material B1 to visible light within the wavelength range of 400-750nm was within a range of 24-35%, the reflectivity to blue light within the wavelength range of 400-500nm was less than or equal to 35%, and the reflectivity to red light within the range of 600-750nm was less than or equal to 27%. Hydrogen gas having a H₂ concentration of 10vol.% was introduced at the flow rate of 100mL/min at room temperature for 60s, the reflection spectrum detection analysis was performed on the hydrogen leakage detecting material DB1 at the moment, the reflectivity range of the hydrogen leakage detecting material DB1 to visible light within the wavelength range of 400-750nm was 22-31%, the reflectivity range of the hydrogen leakage detecting material DB1 to blue light within the wavelength range of 400-500nm was 26-31%, and the reflectivity range of the hydrogen leakage detecting material DB1 to red light within the range of 600-750nm was 22-25%. The highest value of the reflectivity change difference of the hydrogen leakage detection material DB1 to visible light within the wavelength range of 400-750nm before and after introducing H₂ was less than 7%, the highest value of the reflectivity change difference of the hydrogen leakage detection material DB1 to blue light within the wavelength range of 400-500nm before and after introducing H₂ was less than 7%, the highest value of the reflectivity change difference of the hydrogen leakage detection material DB1 to red light within the wavelength range of 600-750nm before and after introducing H₂ was less than 2%, the reflectivity change was not obvious.

The results of TPR analysis, the results of dispersity analysis, and the results of partial hydrogen leakage detection on the hydrogen leakage detecting material DB1 were shown in FIG. 17 and Table 6. As shown in FIG. 17, the starting reduction temperature of the hydrogen leakage detecting material DB1 on hydrogen gas was 250°C.

Hydrogen leakage detection was performed on the hydrogen leakage detecting material DB1, the relevant detection results were as shown in Table 3, Table 4, and Table 5.

### Comparative Example 4

The preparation method of Comparative Example 4 was the same as that of Example 5, except that the reducing reagent was not added. The hydrogen leakage detection control material DB2 was prepared.

Color analysis was performed on the hydrogen leakage detecting material DB2. Since the reducing reagent wan not added, the obtained hydrogen leakage detecting material DB2 had a color substantially consistent with the color-changing substrate A2. Hydrogen gas having a H₂ concentration of 10vol.% was introduced at a flow rate of 100mL/min at room temperature for 60s, hydrogen leakage detecting material DB2 did not exhibit color change compared with the color before introducing hydrogen gas. The results of TPR analysis, the results of dispersity analysis, and the results of partial hydrogen leakage detection on the hydrogen leakage detecting material DB2 were shown in Table 6.

Hydrogen leakage detection was performed on the hydrogen leakage detecting material DB2, the relevant detection results were as shown in Table 3, Table 4, and Table 5.

### Comparative Example 5

The preparation method of Comparative Example 5 was the same as that of Example 5, except that in step (2): 12mL of pure ethylene glycol was measured and taken, the adding mass of the ethylene glycol was 3 times of the adding mass of tungsten oxide. The ethylene glycol was into the solution in step (1) and continuously stirred for 20 min, and subjected to standing still at room temperature for 4 h. The hydrogen leakage detecting material DB3 was prepared.

Color analysis was performed on the hydrogen leakage detecting material DB2. Because the heating operation was not carried out, the color of the obtained hydrogen leakage detecting material DB3 after standing still at room temperature for 4h was basically consistent with that of the color-changing substrate A2. Hydrogen gas having a H₂ concentration of 10vol.% was introduced at a flow rate of 100mL/min at room temperature for 60s, the hydrogen leakage detecting material DB3 did not exhibit color change compared with the color before introducing hydrogen gas.

The results of TPR analysis, the results of dispersity analysis, and the results of partial hydrogen leakage detection on the hydrogen leakage detecting material DB3 were shown in Table 6.

Hydrogen leakage detection was performed on the hydrogen leakage detecting material DB3, the relevant detection results were as shown in Table 3, Table 4, and Table 5.

### Comparative Example 6

The preparation method of Comparative Example 6 was the same as that of Example 5, except that commercially available tungsten trioxide (purchased from the Sinopharm Chemical Reagent Co., Ltd., analytically pure reagent, the crystalline phase was monoclinic phase) was used to prepare the hydrogen leakage detecting material DB4.

The results of TPR analysis, the results of dispersity analysis, and the results of partial hydrogen leakage detection on the hydrogen leakage detecting material DB1 were shown in Table 6 and FIG. 18.

Reflection spectrum detection analysis was performed on the hydrogen leakage detecting material DB4 (before introducing hydrogen gas). Before hydrogen gas was introduced to the hydrogen leakage detecting material DB4, the reflectivity of the hydrogen leakage detecting material DB4 to visible light within the wavelength range of 400-750nm was within a range of 27-34%, the reflectivity to blue light within the wavelength range of 400-500nm was less than or equal to 34%, and the reflectivity to red light within the range of 600-750nm was less than or equal to 28%. Hydrogen gas having a H₂ concentration of 10vol.% was introduced at the flow rate of 100mL/min at room temperature for 900s, the reflectivity range of the hydrogen leakage detecting material DB4 to visible light within the wavelength range of 400-750nm was 25-33%, the reflectivity range of the hydrogen leakage detecting material DB4 to blue light within the wavelength range of 400-500nm was 29-33%, and the reflectivity range of the hydrogen leakage detecting material DB4 to red light within the range of 600-750nm was 25-27%. The highest value of the reflectivity change difference of the hydrogen leakage detection material DB4 to visible light within the wavelength range of 400-750nm before and after introducing H₂ was less than 5%, the highest value of the reflectivity change difference of the hydrogen leakage detection material DB4 to blue light within the wavelength range of 400-500nm before and after introducing H₂ was less than 5%, the highest value of the reflectivity change difference of the hydrogen leakage detection material DB4 to red light within the wavelength range of 600-750nm before and after introducing H₂ was less than 3%. FIG. 18 illustrated the photographs before and after introducing hydrogen gas into the hydrogen leakage detecting material DB4 prepared in Comparative Example 6. After hydrogen gas having a H₂ concentration of 10vol.% of was introduced at a flow rate of 200 mL/min at room temperature for 900s, the hydrogen leak material DB1 before and after introducing hydrogen gas was subjected to the relevant analysis (the reflectivity change difference was less than 10%). As can be seen, the hydrogen leakage detecting material DB1 was yellow before introducing H₂, and the material was also yellow after introducing H₂, thus the color difference was not obvious.

Hydrogen leakage detection was performed on the hydrogen leakage detecting material DB4, the relevant detection results were as shown in Table 3, Table 4, and Table 5.

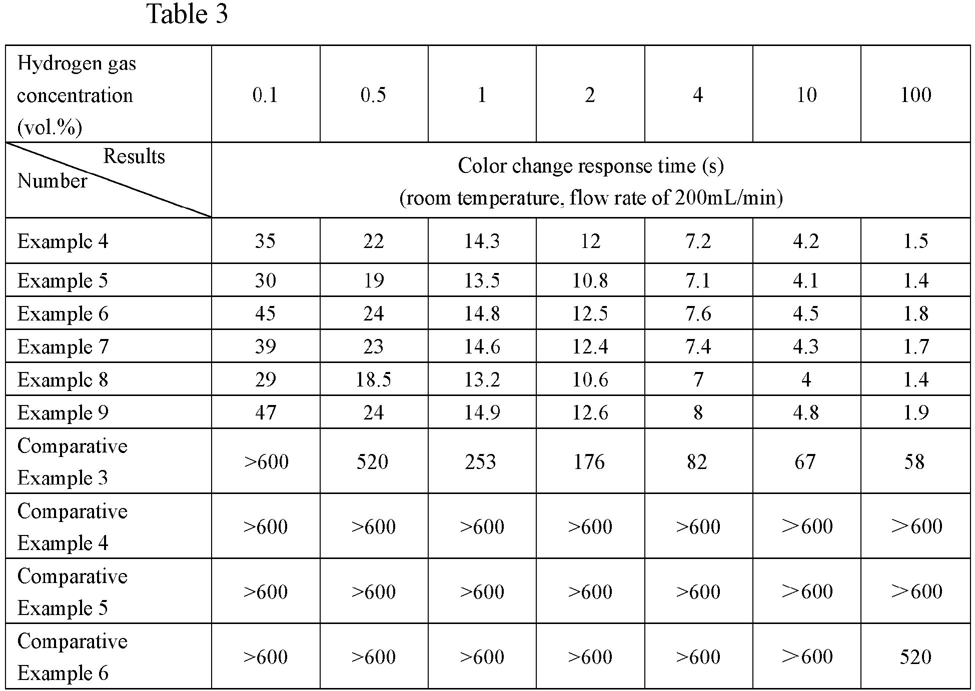

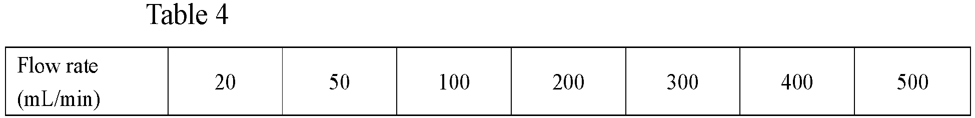

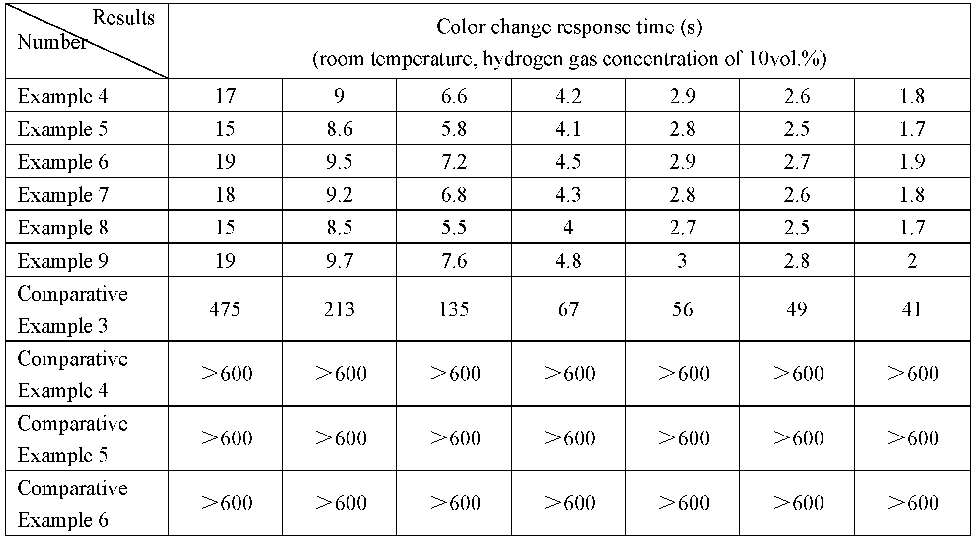

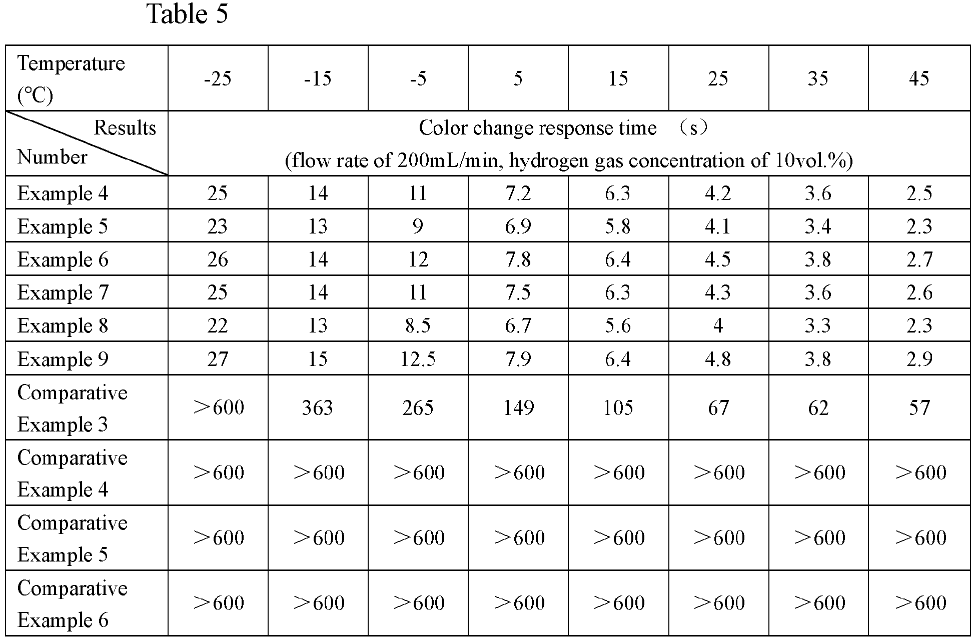

**Table 6**

| | Dispersity (%) | Temperature* (°C) | Time** (s) | Time*** (s) |
|---|---|---|---|---|
| Example 4 | 76 | 46 | 7.2 | 14.3 |
| Example 5 | 77 | 44 | 7.1 | 13.5 |
| Example 6 | 71 | 47 | 7.6 | 14.8 |
| Example 7 | 73 | 45 | 7.4 | 14.6 |
| Example 8 | 80 | 40 | 7 | 13.2 |
| Example 9 | 70 | 52 | 8 | 14.9 |
| Comparative Example 3 | 45 | 250 | 82 | 253 |
| Comparative Example 4 | - | 490 | >600 | >600 |
| Comparative Example 5 | - | 492 | >600 | >600 |
| Comparative Example 6 | 11 | 400 | >600 | >600 |

| | | | | |
|---|---|---|---|---|
| Note: * Starting reduction temperature for hydrogen gas; ** Color-changing response time to hydrogen gas having a concentration of 4vol.% (room temperature, flow rate of 200mL/min); *** Color-changing response time to hydrogen gas having a concentration of 1vol.% (room temperature, flow rate of 200mL/min);. | | | | |

As can be seen from Table 6, the platinum nanoparticles on the hydrogen leakage detecting materials B1-B6 prepared in Examples 4-9 are highly dispersed on tungsten trioxide, the dispersity of the platinum nanoparticles is larger than or equal to 70%, which assists the hydrogen leakage detecting materials to exert better hydrogen adsorption and dissociation activity, especially the low-temperature hydrogen adsorption and dissociation activity, the starting reduction temperature (°C) of each of the hydrogen leakage detecting materials for hydrogen gas is less than 60°C, the color-changing response time for hydrogen gas having a H₂ concentration of 4 vol.% is less than or equal to 10s, the color-changing response time for hydrogen gas having a H₂ concentration of 1 vol.% is less than or equal to 15s, both are far less than the color-changing response time of the hydrogen leakage detecting materials DB1-DB4 prepared in Comparative Examples 3-6, it also demonstrates that the hydrogen leakage detecting material B1-B6 have high sensitivity to hydrogen gas. Therefore, the color change time of the hydrogen leakage detecting materials prepared according to the method of the present disclosure for different hydrogen gas concentrations is significantly shortened.

As illustrated by Example 4, FIG. 8 and FIG. 9, the reflectivity change difference before and after contacting the hydrogen leakage detecting material prepared by the present disclosure with hydrogen gas is large, and the color difference is obvious, it demonstrates that the hydrogen leakage detecting material has high response efficiency to hydrogen gas, the hydrogen leakage detecting material is more beneficial for human eyes to identify the hydrogen leakage sites. Meanwhile, as shown by FIGS. 11-13 and Tables 3-5, the hydrogen leakage detecting material prepared by the present disclosure has a short color-changing response time for hydrogen gas with different hydrogen gas concentrations, different flow rates and different temperatures, and the color-changing response time does not exceed 1min under the conditions such as low hydrogen gas concentration (as low as 0.1 vol.%), high flow rate (as high as 500 mL/min), or low temperature (as low as -25°C).

As illustrated by Example 4, Comparative Example 3, FIG. 2, FIG. 8, FIG. 9 and FIG. 17, the hydrogen depletion detecting material prepared using a hexagonal-phase tungsten trioxide having a nanorod bundle structure as the color-changing substrate has a lower starting reduction temperature for hydrogen gas, and is more sensitive to hydrogen gas at different temperatures.

As shown by Table 6, when the addition amount of the noble metal is within a preferred range of 0.5-2wt.%, the prepared hydrogen leakage detecting material has a high dispersity of the noble metal, which is more than 70%, and the starting reduction temperature for hydrogen gas is lower, and the hydrogen sensitive color-change performance is excellent.

As can be seen from Examples 4 and 8, when the organic liquid phase reducing reagent is a composition of an organic alcohol and a first organic acid, the dispersity of the normal metallic nanoparticles in the prepared hydrogen leakage detecting material is higher (larger than or equal to 80%), the starting reduction temperature for hydrogen gas is lower (less than or equal to 43°C), and the detection sensitivity of the hydrogen leakage detecting material is better.

The above content describes in detail the preferred embodiments of the present disclosure, but the present disclosure is not limited thereto. A variety of simple modifications can be made in regard to the technical solutions of the present disclosure within the scope of the technical concept of the present disclosure, including a combination of individual technical features in any other suitable manner, such simple modifications and combinations thereof shall also be regarded as the content disclosed by the present disclosure, each of them falls into the protection scope of the present disclosure.

## Claims

1. A hydrogen leakage detecting material comprising a color-changing substrate and an active component,
wherein the color-changing substrate is hexagonal-phase tungsten trioxide, and has a microstructure of a nanorod bundle formed by stacking multiple nanorods; wherein the active component is noble metal nanoparticles; wherein a starting reduction temperature of the hydrogen leakage detecting material on hydrogen gas is less than or equal to 100°C.

2. The hydrogen leakage detecting material according to claim 1, wherein the nanorod has a diameter within a range of 1-10nm; the length of the nanorod bundle is within a range of 500-1,000nm, the diameter is within a range of 50-100nm; the nanorod bundle contains 20 or more nanorods, and the length-diameter ratio of the nanorod bundle is within a range of 5-20.

3. The hydrogen leakage detecting material according to claim 1 or 2, wherein the hexagonal-phase tungsten trioxide has main exposed crystal planes (002), (100) and (001), the XRD characteristic diffraction peak intensities satisfy the following conditions: I₍₀₀₂₎=0.9-1.1×I₍₁₀₀₎, and I₍₀₀₂₎=1.2-2×I₍₀₀₁₎, wherein I₍₀₀₂₎ denotes the intensity of the characteristic diffraction peak corresponding to the (002) crystal plane, I₍₁₀₀₎ denotes the intensity of the characteristic diffraction peak corresponding to the (100) crystal plane, and I₍₀₀₁₎ denotes the intensity of the diffraction peak corresponding to the (001) crystal plane.

4. The hydrogen leakage detecting material according to any one of claims 1-3, wherein the reflectivity of said hexagonal-phase tungsten trioxide to visible light within a wavelength range of 400-750nm as measured by a spectrometer is larger than or equal to 50%, preferably larger than or equal to 60%, more preferably larger than or equal to 65%, when using a standard that the reflectivity of barium sulfate is equal to 100%;
preferably, the hexagonal-phase tungsten trioxide has a reflectivity to the blue light within a wavelength range of 400-500nm being larger than or equal to 50%, and a reflectivity to the red light within a wavelength range of 600-750nm being larger than or equal to 70%;
preferably, the hexagonal-phase tungsten trioxide has a reflectivity to the blue light within a wavelength range of 400-500nm being larger than or equal to 67%, and a reflectivity to the red light within a wavelength range of 600-750nm being larger than or equal to 72%.

5. The hydrogen leakage detecting material according to any one of claims 1-4, wherein the noble metal nanoparticles are at least one selected from the group consisting of platinum nanoparticles, palladium nanoparticles, rhodium nanoparticles and gold nanoparticles, preferably platinum nanoparticles.

6. The hydrogen leakage detecting material according to any one of claims 1-5, wherein the dispersity of said noble metal nanoparticles on the color-changing substrate is larger than or equal to 70%, preferably larger than or equal to 75%, more preferably larger than or equal to 80%;
preferably, the average size of the noble metal nanoparticles is less than or equal to 5nm, more preferably less than or equal to 2nm.

7. The hydrogen leakage detecting material according to any one of claims 1-6, wherein the noble metal nanoparticles are contained in an amount of 0.1-1.5wt.%, based on the total mass of the hydrogen leakage detecting material.

8. The hydrogen leakage detecting material according to any one of claims 1-7, wherein a starting reduction temperature of the hydrogen leakage detecting material on hydrogen gas is less than or equal to 80°C, preferably less than or equal to 60°C.

9. The hydrogen leakage detecting material according to any one of claims 1-8, wherein the reflectivity of said hexagonal-phase tungsten trioxide to visible light within a wavelength range of 400-750nm as measured by a spectrometer is larger than or equal to 40%, preferably larger than or equal to 45%, when using a standard that the reflectivity of barium sulfate is equal to 100%;
preferably, the hexagonal-phase tungsten trioxide has a reflectivity to blue light within a wavelength range of 400-500nm as measured by a spectrometer being larger than or equal to 45%, and a reflectivity to red light within a wavelength range of 600-750nm being larger than or equal to 49%, when using a standard that the reflectivity of barium sulfate is equal to 100%.

10. The hydrogen leakage detecting material according to claim 9, wherein a test sample is obtained after contacting the hydrogen leakage detecting material with hydrogen gas having a concentration of 10vol.% for 60s;
the reflectivity change difference of the test sample to visible light within the wavelength range of 400-750nm is larger than or equal to 20%;
preferably, the test sample has a reflectivity change difference to blue light within the wavelength range of 400-500nm being larger than or equal to 22%, and a reflectivity change difference to red light within the wavelength range of 600-750nm being larger than or equal to 30%.

11. The hydrogen leakage detecting material according to any one of claims 1-10, wherein the hydrogen leakage detecting material has a color-changing response time of less than or equal to 2s for pure hydrogen gas, a color-changing response time of less than or equal to 5s for hydrogen gas having a concentration of 10vol.%, a color-changing response time of less than or equal to 10s for hydrogen gas having a concentration of 4vol.%, a color-changing response time of less than or equal to 13s for hydrogen gas having a concentration of 2 vol.%, a color-changing response time of less than or equal to 15s for hydrogen gas having a concentration of 1 vol. %, a color-changing response time of less than or equal to 25s for hydrogen gas having a concentration of 0.5vol.%, and a color-changing response time of less than or equal to 50s for hydrogen gas having a concentration of 0.1vol.%.

12. The hydrogen leakage detecting material according to any one of claims 1-10, wherein the hydrogen leakage detecting material has a color-changing response time of less than or equal to 2.5s at room temperature for hydrogen gas at a flow rate of 500mL/min, a color-changing response time of less than or equal to 3s for hydrogen gas at a flow rate of 400mL/min, a color-changing response time of less than or equal to 4s for hydrogen gas at a flow rate of 300mL/min, a color-changing response time of less than or equal to 5s for hydrogen gas at a flow rate of 200mL/min, a color-changing response time of less than or equal to 8s for hydrogen gas at a flow rate of 100mL/min, a color-changing response time of less than or equal to 10s for hydrogen gas at a flow rate of 50mL/min, a color-changing response time of less than or equal to 20s for hydrogen gas at a flow rate of 20mL/min.

13. The hydrogen leakage detecting material according to any one of claims 1-10, wherein the hydrogen leakage detecting material has a color-changing response time of less than or equal to 27s for hydrogen gas having a concentration of 10vol.% at the temperature of -25°C, a color-changing response time of less than or equal to 15s for hydrogen gas at the temperature of -15°C, a color-changing response time of less than or equal to 13s for hydrogen gas at the temperature of -5°C, a color-changing response time of less than or equal to 8s for hydrogen gas at the temperature of 5°C, a color-changing response time of less than or equal to 6.5s for hydrogen gas at the temperature of 15°C, a color-changing response time of less than or equal to 5s for hydrogen gas at the temperature of 25°C, a color-changing response time of less than or equal to 4s for hydrogen gas at the temperature of 35°C, a color-changing response time of less than or equal to 3s for hydrogen gas at the temperature of 45°C.

14. A method of preparing the hydrogen leakage detecting material according to any one of claims 1-13 comprising the following steps:
(i) carrying out a first mixing for a noble metal precursor, tungsten trioxide and deionized water to obtain a solid-liquid mixture;
(ii) subjecting an organic liquid phase reduction reagent and the solid-liquid mixture to a second mixing to obtain a reaction mixture;
(iii) subjecting the reaction mixture to an organic liquid phase reduction;
wherein the tungsten trioxide is hexagonal-phase tungsten trioxide, and has a microstructure of a nanorod bundle formed by stacking multiple nanorods.

15. The method according to claim 14, wherein the addition amount of the noble metal precursor is 0.2-5 wt.%, preferably 0.5-2wt.%, relative to the addition amount of the tungsten trioxide;
and/or, the addition mass of the organic liquid phase reducing reagent is 1.5-12 times of the addition mass of the tungsten trioxide.

16. The method according to claim 14 or 15, wherein the noble metal precursor is a noble metal soluble salt, preferably a soluble salt of at least one selected from the group consisting of platinum, palladium, rhodium and gold, more preferably at least one selected from the group consisting of potassium hexachloroplatinate, platinum(II) nitrate, chloroplatinic acid, sodium tetrachloroplatinate(II), potassium tetranitroplatinate(II), ammonium hexachloroplatinate(IV) and potassium tetrachloroplatinate(II);
preferably, the organic liquid phase reducing reagent is an organic alcohol and/or a first organic acid, wherein the first organic acid is at least one selected from the group consisting of ascorbic acid, oxalic acid and citric acid, and the organic alcohol is at least one selected from the group consisting of ethylene glycol, propylene glycol and butylene glycol;
preferably, the organic liquid phase reducing reagent is a composition of an organic alcohol and a first organic acid, and the mass ratio of the organic alcohol to the first organic acid is 1: 0.1-5.

17. The method according to any one of claims 14-16, wherein the conditions of the organic liquid phase reduction in step (iii) comprise: the reduction temperature within a range of 105-180°C, preferably within a range of 115-160°C, and the reduction time within a range of 0.5-8h, preferably within a range of 1.5-6 h.

18. Use of the hydrogen leakage detecting material according to any one of claims 1-13 in the hydrogen-containing reducing gas leakage detection product.

19. The use according to claim 18, wherein the hydrogen-containing reducing gas leakage detection product is a hydrogen leakage detection product.

20. The use according to claim 18 or 19, wherein the hydrogen leakage detection product is at least one selected from the group consisting of a hydrogen leakage detection sensor, a hydrogen leakage detection tape, a hydrogen leakage detection coating, a hydrogen leakage detection component, a hydrogen leakage detection film, and a hydrogen leakage detection ink.
